# EUROPEAN PATENT SPECIFICATION

(11) **EP 3 684 790 B1**
(45) Date of publication and mention of the grant of the patent: **28.10.2026**
(21) Application number: 18773599.8
(22) Date of filing: 18.09.2018
(51) Int. Cl.: C07K 14/47, C07K 14/145, C12N 15/64, C12N 15/867

(54) **RETROVIRAL VECTORS**
RETROVIRALE VEKTOREN
VECTEURS RÉTROVIRAUX

(30) Priority: 19.09.2017 GB 201715052
(43) Date of publication of application: 29.07.2020
(73) Proprietor: Oxford Genetics Limited, London, EC4A 3TW (GB)
(72) Inventor: CAWOOD, Ryan, London, EC4A 3TW (GB); PAYNE, Tom, London, EC4A 3TW (GB); DUNAJOVA, Lucia, London, EC4A 3TW (GB); PARKER-MANUEL, Richard, London, EC4A 3TW (GB)
(74) Representative: Dehns
(86) International application number: PCT/GB2018/052656
(87) International publication number: WO 2019/058108

(56) References cited:
- WO-A1-2016/189326
- WO-A2-2006/089001
- COFFIN JM, HUGHES SH, VARMUS HE: "Principles of Retroviral Vector Design", 31 December 1997 (1997-12-31), XP002785617, Retrieved from the Internet <URL:https://www.ncbi.nlm.nih.gov/books/NBK19423/?report=printable> [retrieved on 20181011]
- IKEDA Y ET AL: "Continuous high-titer HIV-1 vector production", NATURE BIOTECHNOLOGY, GALE GROUP INC, vol. 21, no. 5, 1 May 2003 (2003-05-01), pages 569 - 572, XP002263324, ISSN: 1087-0156, DOI: 10.1038/NBT815
- S K MAURYA ET AL: "Retroviral vectors and gene therapy: An update", INDIAN JOURNAL OF BIOTECHNOLOGY, 1 October 2009 (2009-10-01), pages 349 - 357, XP055514739, Retrieved from the Internet <URL:https://pdfs.semanticscholar.org/4f41/e25542b04fe332d1d07ecac7e6aaa43d9059.pdf> [retrieved on 20181011]

## Description

The present invention relates to retroviral vectors comprising double-stranded nucleic acid molecules comprising viral genes for use in the production of retroviral packaging vectors, and retroviral packaging and producer cell lines. In one embodiment, the nucleic acid molecules comprise viral *env* and *gag-pol* genes wherein the coding sequences of the viral *env* and *gag-pol* genes are in opposing orientations.

Retroviruses (including lentiviruses) are positive sense RNA viruses that undergo a complex life cycle involving the reverse transcription of their genome into deoxyribonucleic acid (DNA), which subsequently becomes integrated into the host cell genome following viral infection. They are capable of inserting their genomes, as DNA, into almost any loci in the genome of target cells and mediating long-term expression of virus genes, with the DNA being copied into each daughter cell when the infected cell divides. They generate their genome as an un-spliced mRNA molecule by using the cellular RNA polymerase for transcription. The virus genome is then transported into the cytoplasm using a virus protein called Rev. The genome is then packaged into virus particles in the cytosol using the virus encoded structural proteins Envelope (Env), Gag and Polymerase (Pol). The retrovirus genome is typically 7-10kb in length; in the case of the commonly studied HIV virus, the genome is 9.7kb in length. It exists in each virus particle at 2 copies per virion.

The retrovirus life cycle, and their structural flexibility, affords a number of biotechnological applications, such as the delivery of DNA into the genome of mammalian cells. Furthermore, retroviruses can be modified to contain non-retrovirus glycoproteins in their surface, endowing retrovirus particles with the cellular tropism of the virus from which the glycoprotein originated. This is particularly important when the natural retrovirus glycoprotein has a limited cellular tropism. An example of this is the GP160 glycoprotein of HIV-1, which has evolved to bind the CD4 receptor and only infects cells bearing this protein on their surface. In the case of HIV-1, virus particles are frequently modified to contain a glycoprotein that is different from the natural glycoprotein in a process called pseudotyping. Most commonly, this is achieved with the glycoprotein from vesicular stomatitis virus (VSV-G) to provide a much broader cell tropism.

When using retroviruses in the laboratory as tools, they are typically modified to form replication-incompetent vectors that can express either one or more transgenes or shRNA molecules, and these modified viruses provide versatile vectors for cellular transgene expression and engineering. The flexibility of the retrovirus packaging process also allows for varying genome sizes to be accommodated: genomes as small as 3kb and as large as 18kb can be packaged, although virus titre can be compromised at these extremes.

Several clinical trials have now been performed using retroviruses (and latterly lentiviruses) to infect stem cells ex *vivo* to express transgenes to be supplemented in the treatment of inherited single-gene disorders, before reintroducing them into patients. This is usually done on an autologous basis, although some stem cells can also be applied as heterologous transplants.

Retro/lentiviruses are also finding important applications in the field of adoptive cell transfer, most notably to allow expression of hybrid 'chimeric antigen receptors' (CAR) within T-cells before cell expansion and reinfusion into patients. The CARs generally have an extracellular antibody structure, and an intracellular structure based on the T-cell receptor, but modified (in 2nd and 3rd generation CARs) to improve the quality of cell stimulation following binding of the outside portion to its antigen. This 'CAR T cell' approach has shown impressive success using lentiviruses encoding CARs recognising CD19 in the clinical treatment of B cell lymphoma, and the first US product licence is expected to be granted to Novartis for their CD19-specific CAR T cell, known as CTL019, in the near future. The field of application is now being expanded to address other molecular targets and other malignancies. Hence, there is an expanding need for large scale lentivirus manufacture, something that is challenging to achieve using existing virus production systems.

Alongside clinical use, many laboratories frequently use lentivirus vectors for research and development, where the insertion of exogenous DNA into the cellular genome is required. The versatility of lentiviruses has allowed them to be used to introduce DNA into a wide range of cell types, including but not limited to, human and mouse stem cells, cancer cells, primary tissue cells (e.g. liver, neurons, and fibroblasts).

The infection of these cells is only made possible by coating, or pseudotyping, the virus with a broad tropism glycoprotein, most commonly the VSV-G surface glycoprotein. This protein enables the infection of cells from almost all organs and across many species, including but not limited to, humans, mice, rats, hamsters, monkeys, rabbits, donkeys and horses, sheep, cows and old world apes.

Although wild-type retro/lentiviruses can replicate in host cells, the retro/lentivirus vectors used for transgene and shRNA expression are typically disabled in a range of ways to remove their ability to replicate and cause disease. This means that in order to grow a batch of infectious virus particles which are capable of a single infection round, for experimental or clinical use, it is necessary to provide several virus genes (and thereby virus proteins) that have been genetically removed from the virus genome at the same time into the cells used for virus packaging. These genes are generally provided in three or four separate plasmids, and co-transfected into cells. The central component is a plasmid encoding the virus vector genome (including any transgenes and associated promoters to regulate transcription in target cells) containing packaging signals to direct the assembling virus particles to incorporate the corresponding RNA into the new virus particles. The genes for other virus proteins such as Gag-Pol, Tat and Rev are generally provided from other plasmids that are co-transfected; and yet another plasmid provides the glycoprotein to be incorporated into the envelope of newly-formed virus particles that will direct their infectious tropism. The *gag-pol* expression cassette encodes virus capsid and internal structural proteins and polymerase and protease activity. The *rev* gene acts to enhance nuclear export of retro/lentivirus genomes by binding to a specific region of the virus genome called the Rev Response Element (RRE).

The complexity of retrovirus and lentivirus packaging systems has resulted in a number of 'generations', each with increasing safety on the previous system. In the '1st generation' packaging systems, three plasmids were used: one plasmid encoding all of the HIV genes except for the *envelope* gene; a second plasmid to provide a surface glycoprotein (most often VSV-G); and a plasmid containing the virus genome to be packaged. This system has the disadvantage that the plasmid containing the virus genes contained large regions of DNA with homology to the virus genome plasmid, potentially allowing for recombination between plasmids. This could result in infectious virus being produced capable of causing disease. Other problems included the presence of many virus genes that were not needed for the virus production, including VPU, VIF, VPR and Nef.

In the '2nd generation' systems, five of the nine HIV-1 gene coding regions were removed from the system. This method also resulted in a three-plasmid system, with one plasmid containing the *gag-pol* genes and the ancillary genes for Tat and Rev proteins, a second plasmid encoding a glycoprotein (most often VSV-G) and a third plasmid that encoded the virus genome to be packaged. The virus genomes in this system typically contain wild-type 5' Long Terminal Repeats (LTRs) and hence require the *tat* gene for transcriptional activation and genome production. This system had the advantage that the reduction in homology between the virus genome and the packaging plasmids reduced the likelihood of the formation of potentially hazardous replication-competent retrovirus.

In the most recent '3rd generation' lentiviral vector system, four plasmids are used instead of three. By splitting the system into 4 plasmids (3 helper plasmids and 1 containing the vector genome plus transgene), the '3rd generation' system offers a number of advantages (primarily by increasing the number of recombination events required to form replication-competent virus). However, the '3rd generation' systems also have another significant advantage because they have a modified 5-LTR that includes a promoter, and hence transcription of the genome is not dependent on transcriptional activation by the Tat protein - thereby removing the need for Tat to be encoded in the system. They do not contain the Tat protein on any of the plasmids used. The *rev* gene was also placed on an individual plasmid. Therefore, in 3rd generation systems, the four plasmids contain 1: *gag-pol,* 2: a glycoprotein (most frequently VSV-G), 3: *rev,* and 4: a plasmid encoding a self-inactivating lentivirus genome containing the transgene or RNA of interest. With specific reference to the glycoprotein plasmid, several envelope glycoproteins are available and have been used, but the most widely used is the glycoprotein from Vesicular Stomatitis Virus, known as VSV-G.

Some of these lentivirus packaging genes, notably the *VSV-G* and *gag-pol* components, are widely reported to be toxic to mammalian cells (Burns et al., Proc. Natl. Acad. Sci. 90, 8033-8037 (1993); Yee et al., Proc. Natl. Acad. Sci., 90, 9564-9568 (1994); Hoffman et al., J. Gen. Virol, 91, 2782-2793 (2010). This has provided a substantial barrier to the development of stable packaging cells that express many of the required packaging proteins. Accordingly, batches of lentivirus have been prepared by an inefficient process requiring simultaneous expression of all the plasmids in cells by transient transfection. Such transfection methods are expensive, hard to reproduce at large scale, and often lead to contamination of the virus preparation with plasmids and cellular debris.

It is highly desirable to create 'packaging' cell lines for retroviruses and lentiviruses that encode some, or all, of the components required for production of new virus particles within the cellular genome. This could decrease the complexity of the plasmid transfection required for virus packaging and has the major benefit that every cell will be expressing the genes required for virus production. The ability to create cell lines that express virus proteins with a specific stoichiometry relative to each other would be another significant advantage. There have been several attempts to express virus proteins either stably or under conditional or inducible promoters, for example the STAR cells produced by Ikeda et al. (Nature Biotechnology, 21, 560-572 (2003)) used retroviral transduction of codon-optimised HIV Gag, Pol and Rev to achieve continuous expression in packaging cells. However, the titre of virus produced using these cells is typically below the industry standard of 1x10⁷-1x10⁸/ml. The requirement that some genes must also be inducible, or require independent antibiotic selection agents significantly adds to the system's complexity, and makes scaling up for manufacture significantly more challenging. To date, there have been no cell lines produced that stably and constitutively express the most commonly used retrovirus and lentivirus glycoprotein VSV-G due to its reported toxicity.

Having the sequences encoding the Env protein and Gag/Pol proteins on a single plasmid/vector has the advantage of reducing the number of plasmids which are required to produce the virus particles, thus increasing the efficiency of the viral production system. However, this arrangement suffers from the significant disadvantage that it increases the likelihood of the formation of potentially hazardous replication-competent retroviruses. The chances of this occurring are substantially increased if a single mRNA is produced in cells that contains both the Env and Gag-Pol coding sequences in the same 5' to 3' orientation, where both proteins could be produced from the same mRNA molecule.

The inventors have now found that placing the coding sequences for the Env protein on one strand and Gag/Pol proteins on the opposing strand of the same plasmid/vector ensures that there is no possibility of read-through from a promoter which produces a single mRNA coding for Env and Gag-Pol sequences, thus reducing the risk of replication-competent viruses being formed.

It is therefore an object of the current invention to provide nucleic acid molecules and retroviral packaging vectors in particular which can be used to reduce the number of plasmids which are currently required to produce virus particles, thus increasing the overall efficiency of the viral production system.

In one embodiment, the invention provides a retroviral vector comprising a double-stranded nucleic acid molecule comprising:
(a) a first nucleic acid comprising a viral *env* gene; and
(b) a second nucleic acid comprising a viral *gag-pol* gene;
wherein the coding sequences of the first and second nucleic acids are on opposing strands of the double-stranded nucleic acid molecule, wherein the *env and gag-pol* genes are independently operably-associated with first and second inducible promoters, respectively, the first and second promoters having less than 95% nucleotide sequence identity between them, and wherein the double-stranded nucleic acid molecule comprises nucleotide sequences encoding apoptosis inhibitors IAP1 and EBNA5.

In some embodiments, the double-stranded nucleic acid molecule additionally comprises a nucleic acid comprising a *rev* gene. In some embodiments, the double-stranded nucleic acid molecule additionally comprises a nucleic acid comprising a transgene. Preferably, the retroviral vector is a lentiviral vector.

The nucleic acids in the double-stranded nucleic acid molecule may be DNA or RNA, preferably DNA.

As used herein, the term "retroviral vector" refers to a vector or plasmid which is useful in the production of retroviruses. Examples of retroviral vectors include gamma-retroviral vectors (e.g. vectors derived from murine leukaemia viruses) and lentiviral vectors.

Preferably, the retroviral vector is a lentiviral vector. As used herein, the term "lentiviral vector" refers to a vector or plasmid which is useful in the production of lentiviruses. For example, the lentiviral vector may be a packaging vector, an envelope vector or a packaging/envelope vector.

The *env, gag, pol* and *rev* genes are viral genes. More preferably, they are retroviral genes or derived from retroviral genes.

Examples of retroviruses include lentiviruses, alpha-retroviruses, gamma-retroviruses (e.g. murine leukaemia viruses) and foamy-retroviruses. Preferably, the retrovirus is a lentivirus.

Lentiviruses are a subset of the retroviridae family that are increasingly being used for transgene delivery and protein expression, particularly in progenitor cell populations such as haematopoietic stem cells and T cells. Unlike most retroviruses, lentiviruses are able to deliver their genome, or modified forms thereof, independent of the cell cycle, and often achieve higher efficiency of cellular infection in a shorter time frame. This makes them a much more effective viral vector for both research and clinical use.

The lentivirus family consists of 10 viruses at present. These species are divided into five groups including: Bovine lentivirus group (Bovine immunodeficiency virus and Jembrana disease virus), Equine lentivirus group (Equine infectious anaemia virus, Feline lentivirus group, Feline immunodeficiency virus, Puma lentivirus), Ovine/caprine lentivirus group (Caprine arthritis encephalitis virus, Visna/maedi virus), Primate lentivirus group, (Human immunodeficiency virus 1, Human immunodeficiency virus 2, Simian immunodeficiency virus).

In a preferred embodiment, the lentivirus is Human immunodeficiency virus 1, Simian immunodeficiency virus or Equine infectious anaemia virus. In a more preferable embodiment, the lentivirus is Human immunodeficiency virus 1 or Equine infectious anaemia virus.

The *env, gag, pol* and *rev* genes may be from one or more different viruses (e.g. 2, 3 or 4 different viruses). For example, the *env* gene may be from *Rhabdoviridae* (e.g. VSV-G) whilst other the *gal, pol* and *rev* genes may be from HIV-1.

It is recognised by those in the art that the *env, gag, pol* and *rev* genes of retroviruses vary by clade and isolate. The sequences of these genes from all such clades and isolates are encompassed herein, as well as derivatives thereof.

The first nucleic acid comprises an *env* gene. *env* is a gene that encodes the protein which forms the viral envelope. The expression of the *env* gene enables retroviruses to target and attach to specific cell types, and to infiltrate the target cell membrane. Examples of the *env* gene include the HIV-1 *env* gene and derivatives thereof.

In HIV, the *env* gene codes for the gp160 protein which forms a homotrimer, and is cleaved into gp120 and gp41 by the host cell protease, Furin. The HIV-1 *env* nucleotide and amino acid sequences are given in SEQ ID NOs: 1 and 2, respectively.

As used herein, the term "HIV-1 *env* gene" refers preferably to a nucleotide sequence having the sequence given in SEQ ID NO: 1 or a nucleotide sequence encoding SEQ ID NO: 2, or a nucleotide sequence having at least 80%, 85% 90%, 95% or 99% sequence identity thereto and which encodes a gp160 protein which is capable of forming a homotrimer and is capable of being cleaved into gp120 and gp41 polypeptide by the HIV-1 protease.

The viral envelope may be pseudotyped by using an *env* gene from a virus such as Vesicular Stomatitis virus (VSV), e.g. the *VGV-G* gene, or a derivative thereof.

The VSV-G protein is a single-pass membrane glycoprotein. It mediates a broad infectious tropism. The gene is encoded by a 1536 bp open reading frame and produces a protein consisting of 511 amino acids. The protein contains a 16 amino signal peptide at the N-terminus (amino acid sequence: MLSYLIFALAVSPILG, SEQ ID NO: 14) which is cleaved from the mature protein during export through the secretory pathway to the cell surface. The glycoprotein contains an extracellular region of 458 amino acids and a membrane spanning region (transmembrane region) of 21 amino acids followed by an intracellular (cytosolic) C-terminal region of 22 amino acids. The shuttling of VSV-G protein from the endoplasmic reticulum is rapid, and this is achieved by the specific trafficking signals in the C-terminal tail, including a DxE motif (where x is any amino acid) within the broader trafficking signal Tyr-Thr-Asp-Ile-Glu-Met that contains the DxE motif (Sevier et al., Mol. Biol. Cell. 2000 Jan; 11(1): 13-22). The efficiency of export of VSV-G protein may in part contribute to its effectiveness for retrovirus and lentivirus production. The VSV-G receptor is frequently described as a non-specific fusogenic protein; however, is was recently determined the VSV-G binds to the low-density lipid receptor (LDL-R) (Finkelstein et al., Proc. Natl. Acad. Sci. USA 2013;110(18):7306-7311), which explains its broad cellular tropism and broad application in retrovirus and lentivirus pseudotyping.

As used herein, the term *"VGV-G* gene" refers preferably to a nucleotide sequence having the sequence given in SEQ ID NO: 3 or a nucleotide sequence encoding SEQ ID NO: 4, or a nucleotide sequence having at least 80%, 85% 90%, 95% or 99% sequence identity thereto and which encodes a polypeptide which is capable of attaching to the LDL receptor.

The second nucleic acid comprises a *gag-pol* gene. As used herein, the term *"gag-pol"* includes contiguous/overlapping *gag-pol* genes and independent gag and pol genes.

The Gag-Pol protein of lentiviruses is produced as a single poly-protein that encodes a protease that enables the proteolytic cleavage of the Gag-Pol protein into a number of smaller proteins serving a number of virus functions. The HIV-1 Gag protein is produced from the first translated open reading from the 5'-end of the virus genome and contains a sequence known as the frame-shift sequence. This signal causes the translating ribosome to shift back on the mRNA molecule one base during translation approximately every 1 in 20 translation runs. This process produces the Gag-Pol protein. The result is that lentivirus produce Gag and Gag-Pol at an approximate ratio of 1:20. The Gag protein encodes three major structural proteins: p18, p24 and p15. The Pol protein segment also encodes three major proteins called p10 (protease), p66/55 (reverse transcriptase) and p32 (integrase). The protease is responsible for all of the cleavage events required to produce each of these proteins by proteolytic cleavage. However, the protease recognition sequences that define these cleavage events are poorly defined, suggesting that the protease has broad specificity. This is therefore likely to result in the cleavage of proteins that are not virus related. Indeed, the expression of Gag-Pol proteins is reported to be highly toxic to cells because of this (Blanco et al., The Journal of Biochemistry, 278, 2, 1086-1093, 2003).

In some viruses, the coding sequences of the gag and pol genes overlap. The coding sequences of the gag and pol genes may be contiguous, non-contiguous, overlapping or non-overlapping.

Preferably, the *gag-pol* sequence is from a lentivirus. Examples of the *gag, pol and gag-pol* genes include HIV-1 *gag-pol* genes and derivatives thereof.

In HIV-1, the reading frames of the gag and pol genes overlap, i.e. in a *gag-pol* gene. The HIV-1 *gag-pol* nucleotide sequence is given in SEQ ID NO: 5.

As used herein, the term "HIV-1 *gag-pol* gene" refers preferably to a nucleotide sequence having the sequence given in SEQ ID NO: 5, or a nucleotide sequence having at least 80%, 85% 90%, 95% or 99% sequence identity thereto and which encodes matrix, capsid and nucleocapsid proteins, and a reverse transcriptase, integrase, and protease.

In the double-stranded nucleic acid molecules, the polypeptide-coding sequences of the first and second nucleic acids are on opposing strands of the double-stranded nucleic acid molecule. The two strands of double-stranded nucleic acid molecules are often referred to as the sense/anti-sense strands or positive/negative strands. Therefore, if one defines the nucleic acid strand which includes the coding sequence of the *env* gene as the sense or positive strand, the coding sequences for the *gag* and pol genes will be on the antisense or negative strand.

In some embodiments, the double-stranded nucleic acid molecule additionally comprises a nucleic acid comprising a *rev* gene. Rev is a trans-activating protein that is essential to the regulation of HIV-1 protein expression. A nuclear localization signal is encoded in the *rev* gene, which allows the Rev protein to be localized to the nucleus, where it is involved in the export of unspliced and incompletely spliced mRNAs. Rev binds to a region in the lentivirus genome called the Rev Response Element which allows the nuclear export of unspliced, full length genomes, which is essential for lentivirus production. Examples of the *rev* gene include the HIV-1 *rev* gene and derivatives thereof. The HIV-1 *rev* nucleotide and Rev amino acid sequences are given in SEQ ID NOs: 6 and 7, respectively.

As used herein, the term "HIV-1 *rev* gene" refers preferably to a nucleotide sequence having the sequence given in SEQ ID NO: 6 or a nucleotide sequence encoding SEQ ID NO: 7, or a nucleotide sequence having at least 80%, 85% 90%, 95% or 99% sequence identity thereto and which encodes a protein which is capable of binding to the Rev Response Element (RRE).

In some other embodiments, the double-stranded nucleic acid molecule does not comprise a nucleic acid comprising a *rev* gene.

VSV-G is generally cytotoxic to cells. It is capable of inducing cell fusion and the formation of syncytia. Some of the *gag-pol* gene products are also cytotoxic. In particular, the pol gene encodes a protease that cleaves proteins within the cell and leads to cell death.

The expression of one or more apoptosis inhibitors mitigates or prevents apoptosis of the cell which would otherwise have been initiated by the cytotoxicity of the cytotoxic polypeptide(s). The double-stranded nucleic acid additionally comprises nucleotide sequences encoding apoptosis inhibitors IAP1 and EBNA5.

Examples of apoptosis-inhibiting polypeptides include Celovirus GAM1, Adenovirus E4 Orf6, Adenovirus E1B 55K, Adenovirus E1B 19K, Myxomavirus M11L, Cytomegalovirus IE1, Cytomegalovirus IE2, Baculovirus p35, Baculovirus IAP-1, Herpesvirus US3, Herpesvirus Saimiri ORF16, Herpes Simplex 2 LAT ORF 1, Human XIAP, African Swine Fever ASFV-5-HL (LMW-5-HL/A179L), Kaposi's Sarcoma virus KSbcl2, Vaccinia virus SPI-2, Cowpoxvirus CrmA, Epstein Barr virus BHRF1, Epstein Barr virus EBNA-5, Epstein Barr virus BZLF-1, Papillomavirus E6, Human Aven, Human BCL2 and Human BCL-XL. Other examples of RNA apoptosis inhibitors include Herpesvirus LAT and Adenovirus VA1.

The nucleotide sequences of apoptosis inhibitors IAP1 and EBNA5 are given herein as SEQ ID NOs: 11 and 12, respectively. Particularly preferred are double-stranded nucleic acids which additionally comprises a nucleotide sequence encoding an apoptosis inhibitor comprising SEQ ID NO: 11 or 12.

The production of stable cell lines in mammalian culture typically requires a method of selection to promote the growth of cells containing any exogenously-added DNA. Preferably, the double-stranded nucleic acid molecules additionally comprise a selection gene or an antibiotic resistance gene. To this end, a range of genes are known that provide resistance to specific compounds when the DNA encoding them is inserted into a mammalian cell genome.

Preferably, the selection gene is puromycin N-acetyl-transferase (Puro), hygromycin phosphotransferase (Hygro), blasticidin s deaminase (Blast), Neomycin phosphotransferase (Neo), glutathione S-transferase (GS), zeocin resistance gene (Sh ble), or dihydrofolate reductase (DHFR). Each of these genes provides resistance to a small molecule known to be toxic to mammalian cells, or in the case of GS provides a method for cells to generate glutathione in the absence of glutathione in the growth media.

In a preferred embodiment of the invention, the resistance gene is Puro. This gene is particularly effective because many of the cell lines used in common tissue culture are not resistant; this cannot be said for Neo where many, particularly HEK 293 derivatives, are already Neo resistant due to previous genetic manipulations by researchers (e.g. HEK 293T cells). Puro selection also has the advantage of being toxic over a short time window (<72 hours), and hence it allows variables to be tested rapidly and cells that do not harbour the exogenous DNA to be inserted into the genome are rapidly removed from the culture systems. This cannot be said of some other selection methods such as Hygro, where toxicity is much slower onset.

The development of stable cell lines using selection genes (e.g. Puro) requires that the resistance gene must be expressed in the cells. This can be achieved through a variety of methods including, but not limited to, internal ribosome entry sites (IRES), 2A cleavage systems, alternative splicing, and dedicated promoters.

In a preferred embodiment of the invention, the selection gene will be expressed from a dedicated promoter. This promoter will preferably transcribe in human cells at lower levels than the dedicated promoters driving the *VSV-G* or gag-pol genes.

Each of the genes in the double-stranded nucleic acid molecule which encode a polypeptide or RNA is preferably operably-associated with one or more regulatory elements. This ensures that the polypeptide or RNA is expressed at the desired level and at the desired time.

In this context, the term "regulatory elements" includes one or more of an enhancer, promoter, intron, polyA, insulator or terminator.

The genes used in the vectors herein are preferably separated by polyA signals and/or insulators in an effort to keep transcriptional read-through to other genes to a minimum and also to insulate the genes which it is desired to repress (*VSV-G* and *gag-*pol) under normal circumstances from genes which it is desired to be expressed (e.g. TetR and the apoptosis inhibitors).

While some advantages may be obtained by using copies of the same regulatory element (e.g. promoter sequence) with more than one polypeptide or RNA-encoding nucleotide sequence (in terms of their co-ordinated expression), in this context of this invention, it is highly desirable to use different regulatory elements with each polypeptide or RNA-encoding nucleotide sequence.

Preferably, therefore, the *env* and *gag-pol* genes are operably-associated with different regulatory elements, e.g. different promoter, different intron, different polyA, different insulator and/or different terminator sequences.

The degree of nucleotide sequence identity between the *env* promoter and the *gag-pol* promoter is less than 95%, preferably less than 90%, more preferably less than 85%, 80%, 70% or 60%. More preferably, the degree of nucleotide sequence identity between the *env* terminator and the *gag-pol* terminator is less than 95% or less than 90%, more preferably less than 85%, 80%, 70% or 60%. In this way, the risk of homologous recombination between these regulatory elements is reduced.

The *env* and *gag-pol* genes are independently operably-associated with inducible promoter sequences. The apoptosis inhibitor genes will also be operably-associated with one or more promoters; these may be inducible, repressible or constitutive.

The inducible promoters may ones which are inducible with doxycycline, tetracycline, IPTG or lactose. Preferably, the inducible promoter element comprises a plurality of Tet operator sequences to which the Tet repressor protein (TetR) is capable of binding. In the bound state, tight suppression of transcription is obtained. However, in the presence of doxycycline (or less preferably tetracycline), suppression is alleviated, thus allowing the promoter to gain full transcriptional activity. Such an inducible promoter element is preferably placed downstream of another promoter, e.g. the CMV promoter.

The TetR binding site may have a wild-type sequence, many of which are known in the art. Preferably, the TetR binding site has been subject to one or more improvements by incorporating minor sequence changes. A preferred version that may be used in an embodiment of the invention has the sequence: tccctatcagtgatagaga (SEQ ID NO: 8). Alternative versions of the repressor element that bind the TetR protein or derivatives of the TetR protein may also be used in an embodiment of the invention provided that the TetR repressor protein binds to the TetR binding sequence variant used. Some repressor/binding site variants will have higher than wild-type affinity for each other; these are preferable in an embodiment of the invention.

The TetR gene will be integrated into the chromosome of a human (host) cell. The gene may or may not be integrated adjacent to, or in conjunction with, the *env or gag-pol* genes.

In a preferred embodiment, the coding strand of the TetR gene, when placed adjacent to an *env* or *gag-pol* gene, is in the opposing strand of DNA in the reverse orientation 5' to 3' to the coding sequence of the Gag-Pol proteins. In some embodiments, the *TetR* gene is co-expressed with the *gag-pol* gene.

In one embodiment of the invention, the nucleotide sequence of the TetR protein is as given in SEQ ID NO: 9 or a nucleotide sequence having at least 80%, more preferably at least 85%, 90% or 95% sequence identity thereto and which codes for a TetR protein.

In another embodiment of the invention, the amino acid sequence of the TetR protein is as given in SEQ ID NO: 10 or an amino acid sequence having at least 80%, more preferably at least 85%, 90% or 95% sequence identity thereto and which encodes a TetR protein.

In some preferred embodiments, the promoters which are operably-associated with the *env* gene (preferably the *VSV-G* gene) and the *gag-pol* genes are inducible promoters which are commonly inducible (i.e. inducible together).

More preferably, the promoters which are operably associated with the *env* gene (preferably the *VSV-G* gene) and the *gag-pol* genes both comprise TetR binding sites which allow simultaneous repression and co-expression of the *env* gene (preferably the *VSV-G* gene) and the *gag-pol* genes.

Preferably, the apoptosis inhibitor promoters are selected from the group consisting of RSV, CMV, SV40, PGK and ubiquitin promoters.

Each apoptosis inhibitor is preferably driven independently by a different promoter; and each promoter is preferably of a different type (e.g. CMV, SV40, etc.).

It is preferable that each nucleic acid encoding an apoptosis inhibitor is expressed under the control of a promoter that provides the cell with optimal apoptosis inhibition.

In some preferred embodiments, the double-stranded nucleic acid molecule comprises the use of the following promoter-apoptosis inhibitor combinations:
RSV - Human Inhibitor of Apoptosis (IAP) 1
RSV - Epstein Barr EBNA5
SV40 - Human Inhibitor of Apoptosis (IAP) 1
SV40 - Epstein Barr EBNA5
PGK - Human Inhibitor of Apoptosis (IAP) 1
PGK - Epstein Barr EBNA5
Ubiquitin - Human Inhibitor of Apoptosis (IAP) 1
Ubiquitin - Epstein Barr EBNA5

The double-stranded nucleic acid molecule may additionally comprise at least one transgene.

In some embodiments, the *env* and *gag-pol* genes are flanked by site-specific recombination sites, preferably LoxP or FRT sites, more preferably LoxP sites.

The invention particularly provides a retroviral packaging plasmid comprising a retroviral vector of the invention.

Examples of preferred embodiments of the invention include double-stranded nucleic acid molecules comprising the following elements in this order (wherein the *gag-*pol gene is a viral *gag-pol* gene; and Apoptosis inhibitors 1 and 2 are selected from IAP1 and EBNA5):
*TetR* gene (reverse orientation) - (inducible, e.g. Tet repressible promoter) *VSV-G* gene - (constitutive, e.g. EF1a, promoter) Puromycin resistance - *gag-pol* gene (reverse orientation; inducible, e.g. Tet repressible, promoter) - Apoptosis inhibitor 1 - Apoptosis inhibitor 2.
*TetR* gene (reverse orientation) - (inducible, e.g. Tet repressible promoter) *VSV-G* gene - (IRES, promoter) Puromycin resistance - *gag-pol* gene (reverse orientation; inducible, e.g. Tet repressible, promoter) - Apoptosis inhibitor 1 - Apoptosis inhibitor 2.

There are many established algorithms available to align two amino acid or nucleic acid sequences. Typically, one sequence acts as a reference sequence, to which test sequences may be compared. The sequence comparison algorithm calculates the percentage sequence identity for the test sequence(s) relative to the reference sequence, based on the designated program parameters. Alignment of amino acid or nucleic acid sequences for comparison may be conducted, for example, by computer-implemented algorithms (e.g. GAP, BESTFIT, FASTA or TFASTA), or BLAST and BLAST 2.0 algorithms.

Percentage amino acid sequence identities and nucleotide sequence identities may be obtained using the BLAST methods of alignment (Altschul et al. (1997), "Gapped BLAST and PSI-BLAST: a new generation of protein database search programs", Nucleic Acids Res. 25:3389-3402; and http://www.ncbi.nlm.nih.gov/BLAST). Preferably the standard or default alignment parameters are used.

Standard protein-protein BLAST (blastp) may be used for finding similar sequences in protein databases. Like other BLAST programs, blastp is designed to find local regions of similarity. When sequence similarity spans the whole sequence, blastp will also report a global alignment, which is the preferred result for protein identification purposes. Preferably the standard or default alignment parameters are used. In some instances, the "low complexity filter" may be taken off.

BLAST protein searches may also be performed with the BLASTX program, score=50, wordlength=3. To obtain gapped alignments for comparison purposes, Gapped BLAST (in BLAST 2.0) can be utilized as described in Altschul et al. (1997) Nucleic Acids Res. 25: 3389. Alternatively, PSI-BLAST (in BLAST 2.0) can be used to perform an iterated search that detects distant relationships between molecules. (See Altschul et al. (1997) supra). When utilizing BLAST, Gapped BLAST, PSI-BLAST, the default parameters of the respective programs may be used.

With regard to nucleotide sequence comparisons, MEGABLAST, discontiguous-megablast, and blastn may be used to accomplish this goal. Preferably the standard or default alignment parameters are used. MEGABLAST is specifically designed to efficiently find long alignments between very similar sequences. Discontiguous MEGABLAST may be used to find nucleotide sequences which are similar, but not identical, to the nucleic acids of the invention.

The BLAST nucleotide algorithm finds similar sequences by breaking the query into short subsequences called words. The program identifies the exact matches to the query words first (word hits). The BLAST program then extends these word hits in multiple steps to generate the final gapped alignments. In some embodiments, the BLAST nucleotide searches can be performed with the BLASTN program, score=100, wordlength=12.

One of the important parameters governing the sensitivity of BLAST searches is the word size. The most important reason that blastn is more sensitive than MEGABLAST is that it uses a shorter default word size (11). Because of this, blastn is better than MEGABLAST at finding alignments to related nucleotide sequences from other organisms. The word size is adjustable in blastn and can be reduced from the default value to a minimum of 7 to increase search sensitivity.

A more sensitive search can be achieved by using the newly-introduced discontiguous megablast page (www.ncbi.nlm.nih.gov/Web/Newsltr/FallWinter02/blastlab.html). This page uses an algorithm which is similar to that reported by Ma et al. (Bioinformatics. 2002 Mar; 18(3): 440-5). Rather than requiring exact word matches as seeds for alignment extension, discontiguous megablast uses non-contiguous word within a longer window of template. In coding mode, the third base wobbling is taken into consideration by focusing on finding matches at the first and second codon positions while ignoring the mismatches in the third position. Searching in discontiguous MEGABLAST using the same word size is more sensitive and efficient than standard blastn using the same word size. Parameters unique for discontiguous megablast are:
word size: 11 or 12; template: 16, 18, or 21; template type: coding (0), non-coding (1), or both (2).

In some embodiments, the BLASTP 2.5.0+ algorithm may be used (such as that available from the NCBI) using the default parameters.

In other embodiments, a BLAST Global Alignment program may be used (such as that available from the NCBI) using a Needleman-Wunsch alignment of two protein sequences with the gap costs: Existence 11 and Extension 1.

The invention also provides a kit comprising:
(i) a retroviral packaging vector comprising a retroviral vector of the invention, together with one or more of the following:
(ii) a retroviral Transfer Vector comprising a transgene and a retroviral *rev* gene;
(iii) cells of a cell line suitable for the production of virus particles.

The invention also provides a kit comprising:
(i) a retroviral packaging vector comprising a retroviral vector of the invention which additionally comprises a *rev* gene,
together with one or more of the following:
(ii) a retroviral Transfer Vector comprising a transgene;
(iii) cells of a cell line suitable for the production of virus particles.

The retroviral Transfer Vector contains sites (e.g. LTRs) for insertion of a transgene into a cell genome. Preferably, the 5'-LTR includes a promoter in the Transfer Vector, thus obviating the need for the Tat protein.

The kit may also contain materials for purification of the viral particles such as those involved in the density banding and purification of viral particles, e.g. one or more of centrifuge tubes, lodixanol, dialysis buffers and dialysis cassettes.

The invention also provides a mammalian cell comprising a retroviral vector of the invention. The retroviral vector of the invention may be stably integrated into the nuclear genome of the mammalian cell or present within a vector or plasmid within the cell. Preferably, the retroviral vector of the invention is stably integrated into the nuclear genome of the mammalian cell.

The cells may be isolated cells, e.g. they are not situated in a living animal. Examples of mammalian cells include those from any organ or tissue from humans, mice, rats, hamsters, monkeys, rabbits, donkeys, horses, sheep, cows and apes. Preferably, the cells are human cells. The cells may be primary or immortalised cells. Preferred cells include HEK-293, HEK 293T, HEK-293E, HEK-293 FT, HEK-293S, HEK-293SG, HEK-293 FTM, HEK-293SGGD, HEK-293A, MDCK, C127, A549, HeLa, CHO, mouse myeloma, PerC6, 911, and Vero cell lines. HEK-293 cells have been modified to contain the E1A and E1B proteins and this allows the creation of viruses that have a deletion of the E1A and E1B regions to be grown in this cell line by trans-complementation. Similarly, PerC6 and 911 cells contain a similar modification and can also be used. Most preferably, the human cells are HEK293, HEK293T, HEK293A, PerC6 or 911. Other preferred cells include CHO and VERO cells. Preferably, the cells of the invention are capable of inducibly expressing the *env* and *gag-pol* genes.

The invention also provides a retroviral packaging cell, preferably a mammalian cell, more preferably a human cell), comprising a retroviral vector of the invention.

In a further embodiment, therefore, there is provided a process for producing a retroviral packaging cell, the process comprising the steps:
(i) stably integrating a retroviral vector of the invention into a mammalian cell, thereby producing a mammalian cell that expresses retroviral *env* and *gag-pol* genes, and optionally the *rev* gene.

The invention also provides the use of a retroviral packaging cell of the invention in the production of a retrovirus particle.

The invention also provides a process for producing retroviruses, the process comprising the steps:
(a) introducing a retroviral Transfer Vector comprising 5' and 3' retrovirus LTRs and a retrovirus packaging signal and a retroviral *rev* gene into a retroviral packaging cell of the invention, wherein the retroviral packaging cell comprises retroviral *env* and *gag-pol* genes stably integrated into its genome;
(b) culturing the cell under conditions such that retroviruses are assembled and secreted by the cell; and
(c) harvesting packaged retrovirus from the supernatant.

The invention also provides a process for producing retroviruses, the process comprising the steps:
(a) introducing a retroviral Transfer Vector comprising a transgene into a retroviral packaging cell of the invention, wherein the retroviral packaging cell comprises retroviral *env, gag-pol* and *rev* genes stably integrated into its genome;
(b) culturing the cell under conditions such that retroviruses are assembled and secreted by the cell; and
(c) harvesting packaged retrovirus from the supernatant.
Preferably, the viral vectors are replication-defective or replication-incompetent.

As used herein, the term "introducing" one or more vectors into the cell includes transformation, and any form of electroporation, conjugation, infection, transduction or transfection, *inter alia.* Processes for such introduction are well known in the art (e.g. Proc. Natl. Acad. Sci. USA. 1995 Aug 1;92 (16):7297-301). Preferably, the harvested retroviruses are subsequently purified.

### BRIEF DESCRIPTION OF THE FIGURES

Figures 3, 5-7 and 9-10 relate to embodiments of the invention.
Figure 1: Generation of stable cell lines expressing VSV-G + Apoptosis inhibitors.
Figure 2: Expression of Lentiviral particles from VSV-G stable cell lines.
Figure 3: Schematic illustration of the arrangement of the main expression cassettes in the vectors expressing VSV-G and *gag-pol* genes. An inducible promoter is denoted: i and a constitutive one: c. A.I. indicate apoptosis inhibitors.
Figure 4: Schematic diagram illustrating the arrangement of the main expression cassettes. LTR denotes long terminal repeat, RSV denotes Rous sarcoma virus promoter, PGK denotes the human Phosphoglycerate kinase promoter, G418 denotes the G418 (geneticin) resistance gene and Blast denotes the blasticidin resistance gene.
Figure 5: Transient evaluation of Lentiviral vectors for packaging and producer cell line generation.
Figure 6: Viability of the different packaging cell lines during puromycin selection.
Figure 7: Surface VSV-G expression following induction in identified clones of interest as assessed by % FITC positive population.
Figure 8: VSV-G expression constructs for transcriptional read-through assessment.
Figure 9: Analysis of the preferred packaging line (CV170) using different process conditions in the AMBR bioreactor system.
Figure 10: Fold change in viral titre from un-induced to induced producer cell lines.

### EXAMPLES

The present invention is further illustrated by the following Examples, in which parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention. Examples 2-5 and 7-18 relate to embodiments of the invention.

### Example 1: Identification of factors supporting viral protein expression

The viral glycoprotein, VSV-G, from Vesicular Stomatitis Virus, was co-expressed with a panel of apoptosis inhibitors. We were able to identify several molecules which supported the constitutive expression of VSV-G, and were able to establish a stable cell line via antibiotic selection. This data was subsequently used to select the preferred apoptosis inhibitors to be included in the final Lentiviral packaging and producer cell lines, described in the latter Examples.

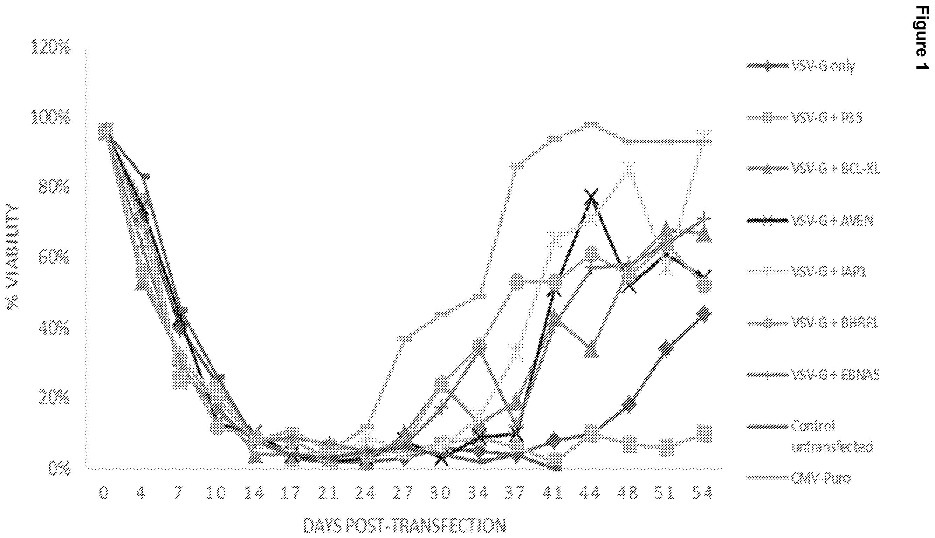

HEK293 suspension cells were transfected using PEI reagent with a range of expression vectors encoding VSV-G operatively linked to a Puromycin selection marker via IRES, as well as a number of apoptosis inhibitor genes. Stable pools were established by media exchange in selective media every 3-4 days. Due to the toxicity of the VSV-G gene, which is constitutively expressed, stable pools took an extended period to establish. The results are shown in Figure 1. Selected apoptosis inhibitors, as illustrated, increased the rate of stable pool recovery, relative to the VSV-G only construct.

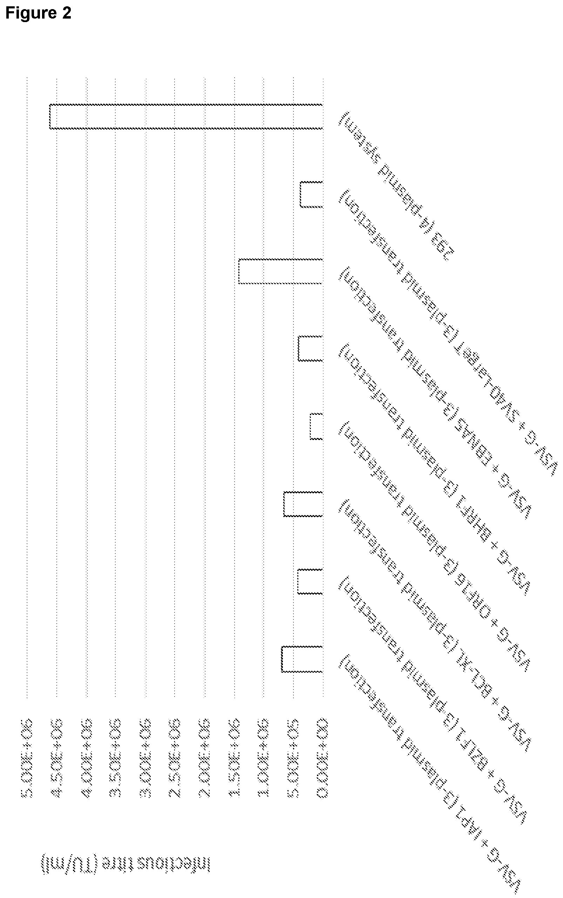

Stable pools were transfected using PEI reagent with 3 vectors encoding all of the factors required for Lentiviral expression (GagPol, Rev, Genome), with the exception of VSV-G which was expressed from the stably-integrated copies. For control purposes, the parental HEK293 line was transfected with the same plasmid set, but also including the VSV-G vector. The genome contained the eGFP gene and this was used for titration (to estimate viral infectious titre) in adherent HEK293 cells. The results are shown in Figure 2. This experiment demonstrated that the constitutive cell lines, when supported by the co-expression of apoptosis inhibitors, were able provide functional VSV-G expression. Such expression is believed to be sufficient in the context of stable producer of packaging cell line to support high-level viral particle production.

### Example 2: Production of constructs for Lentiviral packaging and producer cell line construction

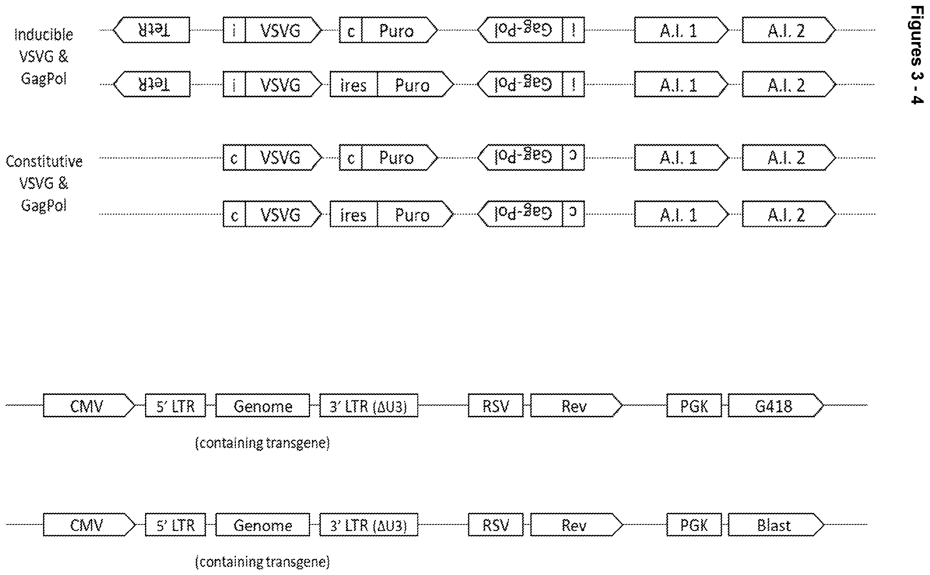

Four constructs were produced as illustrated in Figure 3. The constructs paired *VSV-G* and *gag-pol* genes together using a Puromycin selection marker, and *rev* and genome together using either a G418 or Blasticidin selection marker.

Each cassette can be broken down into functional components as follows:

### VSV-G cassette

- Inducible promoter (PGK CMV fusion promoter converted into an inducible promoter by the addition of two TetO sites in strategic positions) or Constitutive promoter (a PGK CMV fusion promoter)
- 5'UTR = human triose phosphate isomerase (TPI) intron
- CDS (VSV-G codon optimised for expression in HEK293 cells)
- Rabbit beta-globin polyA signal

### GagPol cassette

- Inducible promoter (CMV converted into an inducible promoter by the addition of two TetO sites in strategic positions) or Constitutive promoter (CMV)
- 5'UTR (Human beta-globin intron)
- CDS (wild type HIV1 GagPol)
- RRE from HIV-1
- Human beta-globin polyA signal

### TetR protein expression cassette (Only present in the inducible vectors)

- Promoter (CMV)
- TetR sequence (codon optimised for expression in HEK293 cells)
- PolyA

### Antibiotic resistance marker

- Puro (either IRES-Puro or EF1-alpha-puro)

### Apoptosis inhibitor cassettes

- IAP1 and EBNA5

### Rev/Genome vector

The Rev/genome vector was designed to be stably integrated into the VSV-G/GagPol stable cell line, in order to generate a producer cell line containing all factors required for Lentiviral particle production. A schematic showing the Rev/Genome constructs is shown in Figure 4.

Each of the Rev/Genome vector cassettes can be broken down into functional components as follows:

### Genome

- Third generation lentiviral genome
- Chimeric 5' LTR fused to a heterologous CMV promoter driving transcription of the lentiviral genome.

### Transgene cassette

- The transgene is green fluorescent protein (GFP)or an anti-CD19 chimeric antigen receptor (CAR).
- The transgene is expressed from the Spleen focus forming virus (SFFV) promoter.

### Rev cassette

- Constitutive RSV promoter
- Rev CDS

### Antibiotic resistance marker

- PGK-G418 or PGK-Blast

### Example 3: Analysis of constructs for Lentiviral packaging and producer cell line construction in transient context

In order to evaluate the functionality of the vectors to be used in stable cell lines, they were first used to generate Lentiviral particles in transient experiments in adherent cells. This allowed benchmarking against standard 4-vector transient expression vectors.

Adherent 293T cells were transfected with 2, 3 or 4-plasmid lentiviral packaging systems which included constructs (constitutive or inducible) generated for the purposes of stable lentivirus producer cell line construction. Throughout, eGFP was used as the transgene in the genome vector.

The lentivirus containing supernatant was collected after 72 hours, serially diluted in DMEM and used to infect adherent 293 cells. After 72 hours, the cells were trypsinized and analysed on the flow cytometer for eGFP signal.

Data from serial dilution at which 10-20% eGFP positive (i.e. transduced) cells had been achieved was then used to calculate infectious particle concentration. This was used to gauge the performance of the different packaging/producer constructs under evaluation.

Day 0: 293T cells were seeded into 6-well plates in DMEM supplemented with 10% Foetal Bovine serum at a pre-defined density, ready for transfection the following day. Cells were incubated overnight at 37°C, 5% CO₂ in a humidified incubator.

Day 1: Cells were transfected using branched PEI with the combinations of plasmids as detailed in Table 1 with or without Doxycycline, regardless of whether the constructs under evaluation were constitutive or inducible.

Day 3: 293 cells were seeded into 48-well plates in DMEM + 10% FBS at a pre-defined density, ready for infection the following day. Cells were incubated overnight at 37°C, 5% CO₂ in a humidified incubator.

Day 4: Harvest of lentiviral supernatants followed by centrifugation to remove cellular debris. Lentiviruses were serially diluted and used to infect 293 cells.

Day 7: Harvest of transduced cells and analysis by flow cytometry. The dilution at which 10-20% eGFP positive (i.e. transduced) cells had been achieved was then used to calculate infectious particle concentration.

Combinations of 4, 3 and 2 plasmid system constructs were tested for the production of transient lentivirus in adherent 293T cells. The combinations are shown below in Table 1.

**Table 1:**

| 4 plasmid system (4P) - Constant components GagPol, Rev and eGFP genome on individual plasmids | | | | | | | |
|---|---|---|---|---|---|---|---|
| Varied component VSV-G | | | | | | | |
| **VSVG** | **Apoptosis inhibitor** | | **Constitutive/Inducible** | | | **Promoter** | |
| P6189 | None | | Constitutive | | | | |
| P7847 | None | | Constitutive | | | IRES Puro | |
| P8170 | IAP1 | | Constitutive | | | promoter(EF1a)-Puro | |
| P8176 | BCL-XI | | Constitutive | | | promoter(EF1a)-Puro | |
| P8238 | EBNA5 | | Constitutive | | | IRES Puro | |
| Q2589 | IAP1 | | Inducible | | | | |
| Q2583 | BCL-XI | | Inducible | | | promoter(EF1a)-Puro | |
| Q2586 | EBNA5 | | Inducible | | | promoter(EF1a)-Puro | |

| 3 plasmid system (3P) - Constant components: Rev and eGFP genome on individual plasmids | | | | | | | |
|---|---|---|---|---|---|---|---|
| Varied component VSV-G/GagPol on a single plasmid | | | | | | | |
| **VSV-G/GagPol** | | **Apoptosis inhibitor** | | | **Constitutive/Inducible** | | **Promoter** |
| P6998 | | none | | | Constitutive | | |
| Q1619 | | none | | | Constitutive | | IRES Puro |
| Q1781 | | none | | | Constitutive | | promoter(EF1a)-Puro |
| Q1852 | | IAP1 | | EBNA5 | Constitutive | | promoter(EF1a)-Puro |
| Q1784 | | IAP1 | | EBNA5 | Constitutive | | IRES Puro |
| Q1621 | | none | | | Inducible | | promoter(EF1a)-Puro |
| Q1850 | | IAP1 | | EBNA5 | Inducible | | promoter(EF1a)-Puro |

| 3 plasmid system (3P) - VSV-G and GagPol on individual plasmids, combination Rev and eGFP genome Q1847 | | | | | | | |
|---|---|---|---|---|---|---|---|
| 2 plasmid system (2P) - Constant components: Rev and eGFP genome on a single plasmid | | | | | | | |
| Varied component VSV-G/GagPol on a single plasmid | | | | | | | |
| **VSV-G/GagPol** | **Apoptosis inhibitor** | | **Constitutive/Inducible** | | **Promoter** | | |
| P6998 | none | | Constitutive | | | | |
| Q1619 | none | | Constitutive | | IRES Puro | | |
| Q1781 | none | | Constitutive | | promoter(EF1a)-Puro | | |
| Q1852 | IAP1 | EBNA5 | Constitutive | | promoter(EF1a)-Puro | | |
| Q1784 | IAP1 | EBNA5 | Constitutive | | IRES Puro | | |
| Q1621 | none | | Inducible | | promoter(EF1a)-Puro | | |
| Q1850 | IAP1 | EBNA5 | Inducible | | promoter(EF1a)-Puro | | |

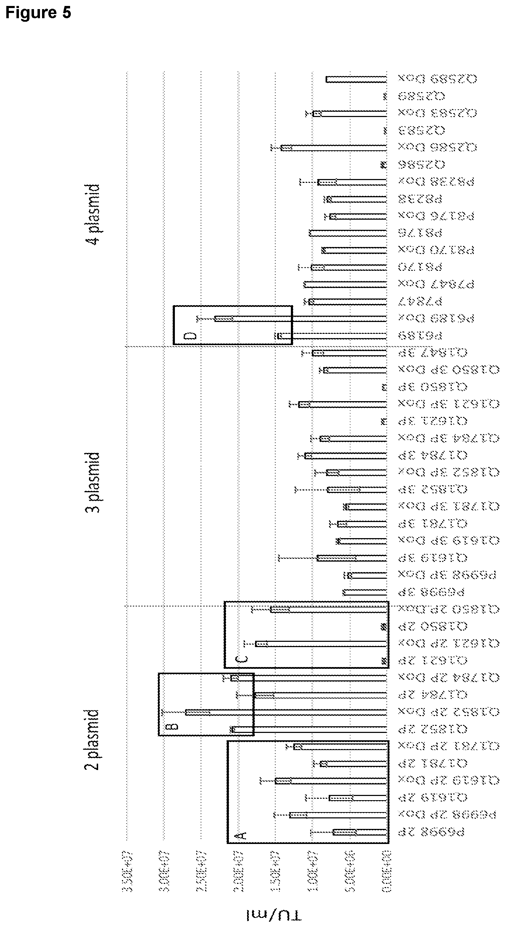

A number of conclusions were drawn from this data:
- All components within the Lentiviral vectors are fully functional.
- Two plasmid systems are capable of giving high Lentiviral titres, and in many cases outperform, or are equivalent to, 3 or 4-plasmid systems.
- Constitutive VSV-G/GagPol constructs also encoding apoptosis inhibitors in the 2-plasmid system showed boosted lentivirus production when compared to the identical constructs lacking these cassettes (Figure 5, Box A versus Box B).
- Inducible VSV-G/GagPol constructs with or without apoptosis inhibitors, showed high-level viral particle expression, only slightly below constitutive constructs (Figure 5, Box C versus Box B).
- Inducible constructs (Box C) show very tight gene regulation, as indicated by expression levels in the absence of Dox.
- Overall, Doxycycline treatment increased viral particle expression, irrespective of the induction system.

### Example 4: Generation and analysis of VSV-G/GagPol packaging cell lines

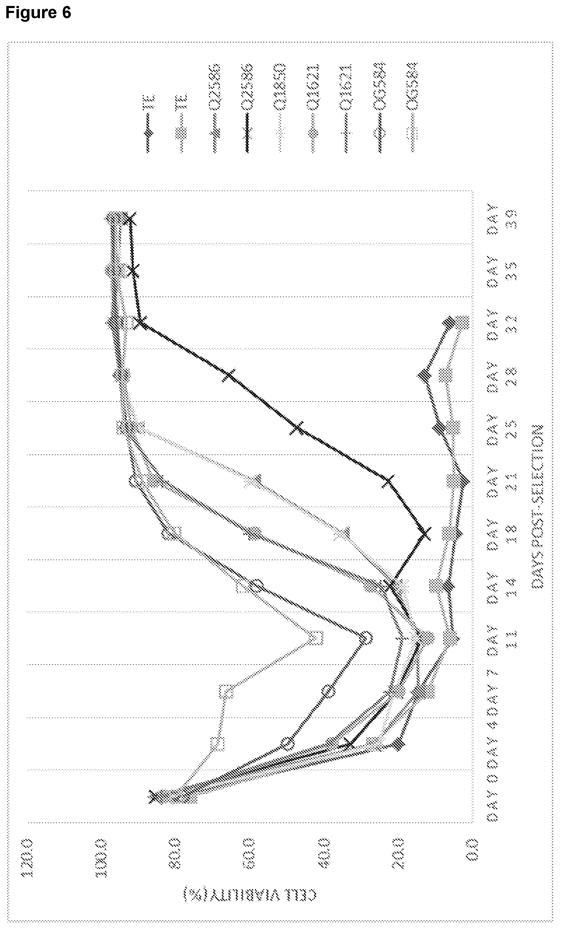

Suspension HEK293 cells were transfected with linearized inducible packaging constructs encoding either VSV-G or VSV-G and GagPol with or without apoptosis inhibitors (detailed in Table 2 below) using linear PEI. Subsequently, cells with stably integrated genes were selected for with puromycin treatment until ≥90% viability was obtained with growth characteristics similar to the host cell line (Figure 1). The VSV-G/GagPol cell pools were carried forward to single cell cloning to obtain a monoclonal high expressing cell line. This was performed via single cell sorting using the Sony SH800 Cell Sorter into prepared cloning media in 96 well plates. Single cells forming colonies were then taken through several scale-up stages until sufficient numbers of cells were obtained for VSV-G protein expression assessment. For this purpose, cell clones were incubated with either vehicle control (DMSO) or Doxycycline for 24 hours to induce VSV-G/GagPol expression. The cells were stained with anti-VSV-G and corresponding secondary FITC-conjugated antibody and analysed by flow cytometry for FITC positive cells. The results are shown in Figure 6.

**Table 2. Constructs used in stable packaging cell line generation**

| **Plasmid** | **Description** | **Apoptosis inhibitor** | | **Constitutive/ Inducible** |
|---|---|---|---|---|
| Q2586 | Inducible VSVG with promoter(EF1a)-Puro + EBNA5 cassette | EBNA5 | | Inducible |
| Q1850 | Inducible VSVG & GagPol with promoter(EF1a)-Puro + IAP1/EBNA5 cassettes | IAP1 | EBNA5 | Inducible |
| Q1621 | Inducible VSVG & GagPol with promoter(EF1a)-Puro | | | Inducible |
| OG584 | CMV-Puromycin | | | |

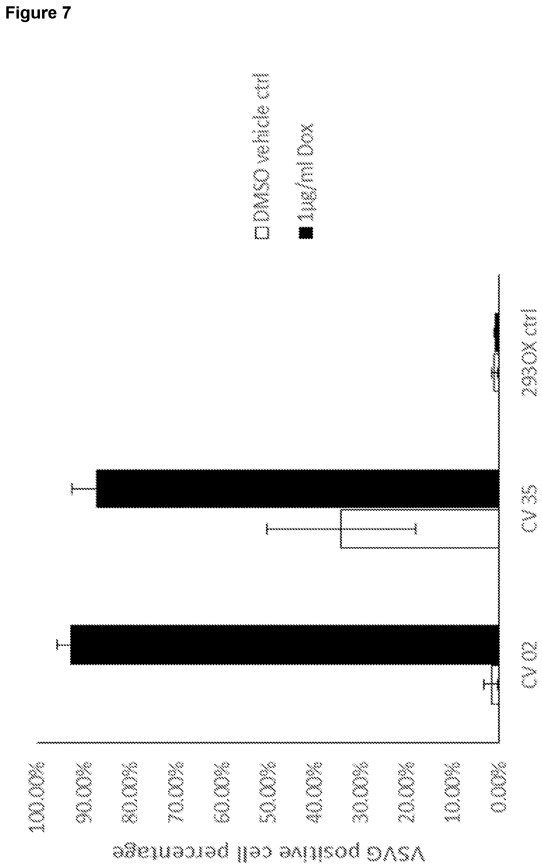

A large number of clonal cell lines were produced following cloning experiments. Representative data from two clones is shown in Figure 7. Both clone CV02 and CV 35 showed high level expression of VSV-G following doxycycline treatment. However, CV 35 also showed high basal expression of VSV-G, indicating leaky-functionality of the Tet regulation system. This demonstrates that using the generated packaging constructs, stable high expression of VSV-G/GagPol genes can be achieved in the inducible context.

### Example 5: Generation and analysis of producer cell lines

Monoclonal VSV-G/GagPol suspension HEK293 cell lines (as detailed in Example 4) are transfected with linearized constitutive constructs encoding either Rev only or a combination of Rev and viral genome (eGFP or CD19) using linear PEI (see Table 3 for construct details). Subsequently, cells with stably integrated genes are selected for with blasticidin selection until ≥90% viability is obtained with growth characteristics similar to the host cell line. The generated producer cells are induced with Doxycycline, the culture supernatant collected after a set production period and clarified from cell debris. Subsequently, this viral supernatant is serially diluted and used for infection of Jurkat cells (eGFP and CD19) or adherent 293 cells (eGFP only) for 72 hours. Jurkat and 293 cells infected with eGFP lentivirus are directly analysed via flow cytometry, whereby the dilution yielding 10-20% eGFP positive (i.e. transduced) cells is used to calculate the infectious particle concentration.

In the instance of CD19, infected Jurkat cells are stained via protein L to detect surface CD19 expression and similarly to eGFP, infectious particle concentrations are estimated. The packaging cell line (VSV-G/GagPol + Rev) is evaluated using simultaneous Doxycycline induction of VSV-G/GagPol expression and transient transfection of eGFP virus genome. The produced viral supernatant is then serially diluted and used to infect Jurkat and 293 cells to establish infectious particle concentrations. The preferred producer pools are taken forward to single cell sorting using the Sony SH800 Cell Sorter into prepared cloning media in 96 well plates. Single cells forming colonies are then taken through several scale-up stages until sufficient numbers of cells are obtained for assessing lentivirus production following doxycycline induction in a manner outlined above.

**Table 3. Constructs used for transfection of the monoclonal packaging cell line for stable producer cell line generation**

| **Plasmids** | **Description** | **Constitutive/Inducible** |
|---|---|---|
| Q3928 | pSF-nano-lenti-genome-SFFV-eGFP-RSV-Rev-PGK-Blast | Constitutive |
| Q3931 | pSF-nano-lenti-genome-SFFV-CD19-CAR-ORIG-RSV-Rev-PGK-Blast | Constitutive |
| Q3939 | pSF-core-RSV-Rev-PGK-Blast-KanR | Constitutive |
| OG588 | pSF-CMV-Blast | Constitutive |

### Example 6: Analysis of transcriptional read through between viral genes and potential to generate replication competent Lentivirus (RCL)

A major safety implication with the use of retroviral vectors is the risk of generating replication-competent particles. This has been addressed in the context of transient Lentivirus expression by third generation systems, which incorporate separate vectors for the packaging factors, as well as a deletion within the LTR regions to ablate promoter activity, making transcription of the transgene dependent on an internal promoter.

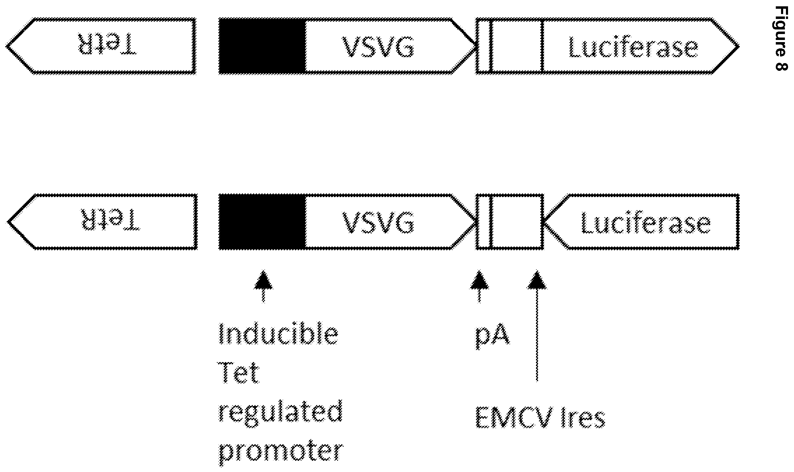

To assess the configuration of the *VSV-G* and *gag-pol* genes in the constructs used to generate the stable bioproduction cell lines, and its potential to reduce risk of RCL generation, two additional constructs were generated where *VSV-G* was expressed in tandem with a Luciferase reporter, in both forward and reverse orientations. This is illustrated in Figure 8.

The two constructs were subsequently transiently transfected into adherent HEK293T cells, treated with Doxycycline to induce transcription, and luciferase activity assayed after 24hrs. Presence of luciferase activity in the forward orientation construct is indicative of read-through transcription, which directly relates to safety concerns that are avoided by the gene configuration used in this invention in stable cell line context.

### Example 7: Analysis of VSV-G/GagPol packaging cell lines

Additional packaging cell lines were produced by identical methods to those described in Example 4. Packaging cell lines were grown in both shake flask and in miniature bioreactors systems (AMBR15), in order to analyse and optimise production parameters. Those illustrated here were produced by stable integration of the linearised Q1850 plasmid.

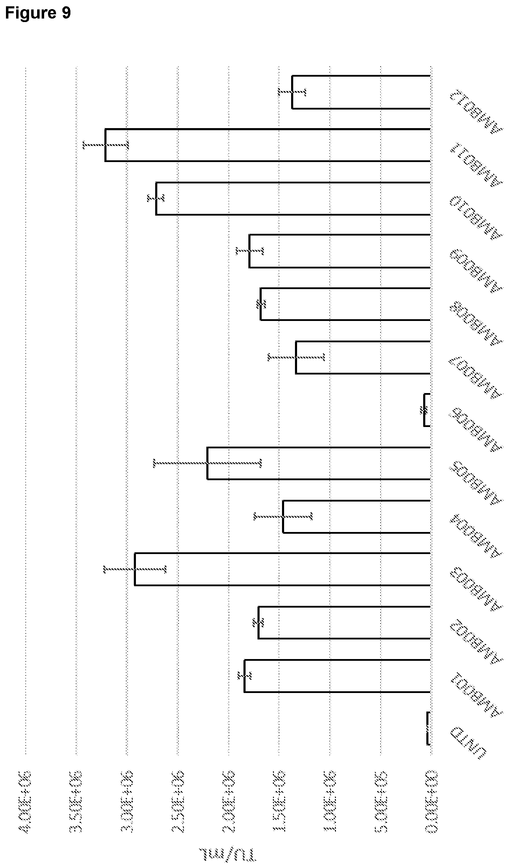

To investigate production parameters, packaging cell lines were cultured for 72hrs after PEl-based transfection of Rev/Genome plasmid (Q1847) and induced using doxycycline. Supernatants were collected and analysed by flow cytometry-based infectious titre assay. Data is presented in Figure 9, and represents analysis of the preferred packaging line (CV170) using different process conditions in the AMBR bioreactor system.

### Example 8: Analysis of producer cell lines

The preferred packaging cell line (CV170) was used as the starting point for generation of the fully stable producer cell line. A stable pool was created by stable integration of the linearised Q3928 plasmid, and then cloned by FACS sorting. Clonal lines were then expanded and analysed in deep well shaking plates. Based on this data, selected clonal cell lines were scaled-up into shake flask culture.

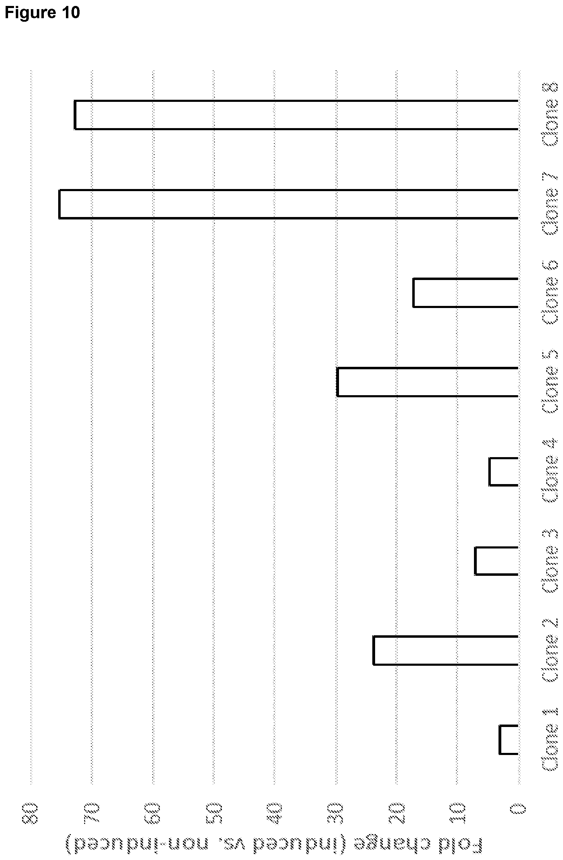

Once transferred into shake flask, producer cell lines were induced by addition of doxycycline to the cell culture media. 72hrs post-induction, supernatants were collected and analysed by flow cytometry-based infectious titre assay. Data is presented in Figure 10 as fold change in viral titre from un-induced to induced.

### SEQUENCES

**SEQ ID NO: 1 - HIV-1 *env* nucleotide** 
ATGAGAGTGAAGGAGAAATATCAGCACTTGTGGAGATGGGGGTGGAGATGGGGCACCATG
CTCCTTGGGATGTTGATGATCTGTAGTGCTACAGAAAAATTGTGGGTCACAGTCTATTAT
GGGGTACCTGTGTGGAAGGAAGCAACCACCACTCTATTTTGTGCATCAGATGCTAAAGCA
TATGATACAGAGGTACATAATGTTTGGGCCACACATGCCTGTGTACCCACAGACCCCAAC
CCACAAGAAGTAGTATTGGTAAATGTGACAGAAAATTTTAACATGTGGAAAAATGACATG
GTAGAACAGATGCATGAGGATATAATCAGTTTATGGGATCAAAGCCTAAAGCCATGTGTA
AAATTAACCCCACTCTGTGTTAGTTTAAAGTGCACTGATTTGAAGAATGATACTAATACC
AATAGTAGTAGCGGGAGAATGATAATGGAGAAAGGAGAGATAAAAAACTGCTCTTTCAAT
ATCAGCACAAGCATAAGAGGTAAGGTGCAGAAAGAATATGCATTTTTTTATAAACTTGAT
ATAATACCAATAGATAATGATACTACCAGCTATAAGTTGACAAGTTGTAACACCTCAGTC
ATTACACAGGCCTGTCCAAAGGTATCCTTTGAGCCAATTCCCATACATTATTGTGCCCCG
GCTGGTTTTGCGATTCTAAAATGTAATAATAAGACGTTCAATGGAACAGGACCATGTACA
AATGTCAGCACAGTACAATGTACACATGGAATTAGGCCAGTAGTATCAACTCAACTGCTG
TTAAATGGCAGTCTAGCAGAAGAAGAGGTAGTAATTAGATCTGTCAATTTCACGGACAAT
GCTAAAACCATAATAGTACAGCTGAACACATCTGTAGAAATTAATTGTACAAGACCCAAC
AACAATACAAGAAAAAGAATCCGTATCCAGAGAGGACCAGGGAGAGCATTTGTTACAATA
GGAAAAATAGGAAATATGAGACAAGCACATTGTAACATTAGTAGAGCAAAATGGAATAAC
ACTTTAAAACAGATAGCTAGCAAATTAAGAGAACAATTTGGAAATAATAAAACAATAATC
TTTAAGCAATCCTCAGGAGGGGACCCAGAAATTGTAACGCACAGTTTTAATTGTGGAGGG
GAATTTTTCTACTGTAATTCAACACAACTGTTTAATAGTACTTGGTTTAATAGTACTTGG
AGTACTGAAGGGTCAAATAACACTGAAGGAAGTGACACAATCACCCTCCCATGCAGAATA
AAACAAATTATAAACATGTGGCAGAAAGTAGGAAAAGCAATGTATGCCCCTCCCATCAGT
 
GGACAAATTAGATGTTCATCAAATATTACAGGGCTGCTATTAACAAGAGATGGTGGTAAT
AGCAACAATGAGTCCGAGATCTTCAGACCTGGAGGAGGAGATATGAGGGACAATTGGAGA
AGTGAATTATATAAATATAAAGTAGTAAAAATTGAACCATTAGGAGTAGCACCCACCAAG
GCAAAGAGAAGAGTGGTGCAGAGAGAAAAAAGAGCAGTGGGAATAGGAGCTTTGTTCCTT
GGGTTCTTGGGAGCAGCAGGAAGCACTATGGGCGCAGCCTCAATGACGCTGACGGTACAG
GCCAGACAATTATTGTCTGGTATAGTGCAGCAGCAGAACAATTTGCTGAGGGCTATTGAG
GCGCAACAGCATCTGTTGCAACTCACAGTCTGGGGCATCAAGCAGCTCCAGGCAAGAATC
CTGGCTGTGGAAAGATACCTAAAGGATCAACAGCTCCTGGGGATTTGGGGTTGCTCTGGA
AAACTCATTTGCACCACTGCTGTGCCTTGGAATGCTAGTTGGAGTAATAAATCTCTGGAA
CAGATTTGGAATCACACGACCTGGATGGAGTGGGACAGAGAAATTAACAATTACACAAGC
TTAATACACTCCTTAATTGAAGAATCGCAAAACCAGCAAGAAAAGAATGAACAAGAATTA
TTGGAATTAGATAAATGGGCAAGTTTGTGGAATTGGTTTAACATAACAAATTGGCTGTGG
TATATAAAATTATTCATAATGATAGTAGGAGGCTTGGTAGGTTTAAGAATAGTTTTTGCT
GTACTTTCTATAGTGAATAGAGTTAGGCAGGGATATTCACCATTATCGTTTCAGACCCAC
CTCCCAACCCCGAGGGGACCCGACAGGCCCGAAGGAATAGAAGAAGAAGGTGGAGAGAGA
GACAGAGACAGATCCATTCGATTAGTGAACGGATCCTTGGCACTTATCTGGGACGATCTG
CGGAGCCTGTGCCTCTTCAGCTACCACCGCTTGAGAGACTTACTCTTGATTGTAACGAGG
ATTGTGGAACTTCTGGGACGCAGGGGGTGGGAAGCCCTCAAATATTGGTGGAATCTCCTA
CAGTATTGGAGTCAGGAACTAAAGAATAGTGCTGTTAGCTTGCTCAATGCCACAGCCATA
GCAGTAGCTGAGGGGACAGATAGGGTTATAGAAGTAGTACAAGGAGCTTGTAGAGCTATT
CGCCACATACCTAGAAGAATAAGACAGGGCTTGGAAAGGATTTTGCTATAA
**SEQ ID NO: 2 - HIV-1 Env amino acid** 
MRVKEKYQHLWRWGWRWGTMLLGMLMICSATEKLWVTVYYGVPVWKEATTTLFCASDAKA
YDTEVHNVWATHACVPTDPNPQEVVLVNVTENFNMWKNDMVEQMHEDIISLWDQSLKPCV
KLTPLCVSLKCTDLKNDTNTNSSSGRMIMEKGEIKNCSFNISTSIRGKVQKEYAFFYKLD
IIPIDNDTTSYKLTSCNTSVITQACPKVSFEPIPIHYCAPAGFAILKCNNKTFNGTGPCT
NVSTVQCTHGIRPVVSTQLLLNGSLAEEEVVIRSVNFTDNAKTIIVQLNTSVEINCTRPN
NNTRKRIRIQRGPGRAFVTIGKIGNMRQAHCNISRAKWNNTLKQIASKLREQFGNNKTII
FKQSSGGDPEIVTHSFNCGGEFFYCNSTQLFNSTWFNSTWSTEGSNNTEGSDTITLPCRI
KQIINMWQKVGKAMYAPPISGQIRCSSNITGLLLTRDGGNSNNESEIFRPGGGDMRDNWR
SELYKYKVVKIEPLGVAPTKAKRRVVQREKRAVGIGALFLGFLGAAGSTMGAASMTLTVQ
ARQLLSGIVQQQNNLLRAIEAQQHLLQLTVWGIKQLQARILAVERYLKDQQLLGIWGCSG
KLICTTAVPWNASWSNKSLEQIWNHTTWMEWDREINNYTSLIHSLIEESQNQQEKNEQEL
LELDKWASLWNWFNITNWLWYIKLFIMIVGGLVGLRIVFAVLSIVNRVRQGYSPLSFQTH
LPTPRGPDRPEGIEEEGGERDRDRSIRLVNGSLALIWDDLRSLCLFSYHRLRDLLLIVTR
IVELLGRRGWEALKYWWNLLQYWSQELKNSAVSLLNATAIAVAEGTDRVIEVVQGACRAI
RHIPRRIRQGLERILL
**SEQ ID NO: 3 *- VSV-G* nucleotide** 
atgaagtgtctgctgtacctggcgttcctgtttatcggggtgaactgcaagttcactatcgtgtttccgcacaacca
aaagggcaactggaaaaacgtgccttcaaattaccattattgccccagcagctcggacctgaactggcacaatgacc
tcattggaaccgcgctgcaggtgaagatgccaaagagccacaaggctatccaggctgacggatggatgtgccacgcg
tcaaaatgggtgactacctgcgatttccgctggtacggaccaaaatacatcacgcacagcatcagatcattcacccc
gtcagtggaacaatgcaaagaatccatcgaacagactaagcagggaacctggctgaaccctggatttccgccgcagt
cgtgtgggtacgcaaccgtgaccgatgcagaggccgtgatcgtgcaagtcacgccgcatcacgtgcttgtggacgag
tacaccggagaatgggtcgattcccagttcatcaacggcaagtgctccaactacatttgcccaaccgtgcacaacag
cactacttggcacagcgactacaaagtgaagggtctgtgtgattccaacctgatctccatggatatcactttcttct
cggaagacggcgaactgtcctcactgggcaaagaaggaactgggtttcgctcaaattacttcgcctacgaaactgga
ggaaaagcctgcaagatgcagtactgcaagcactgggcgtgagactacccagcggtgtctggttcgagatggccga
taaggacctgtttgcagcagcgagattcccggaatgccctgagggatcgagcatctccgctccaagccaaacttcag
tggacgtgagcctgatccaggacgtggaacggattctcgactactcgctgtgccaggagacctggtcgaagatcaga
gcgggactgcccatctcaccggtggacctgtcctacctggcgccaaagaatccgggcactggaccggcgttcaccat
catcaacggcaccctcaaatacttcgagacgcggtacatccgggtggacatcgcagctccgatcctctcccggatgg
tgggaatgatctcggggactactaccgaacgcgagctctgggacgactgggcaccttacgaggatgtcgagatcgga
cctaacggagtgctccggacctcctccgggtacaagttccctctgtacatgatcggccatggcatgctggactcgga
tctgcatctgtcgtccaaagcacaggtgtttgaacacccacacattcaagacgccgccagccagctgccggacgatg
agtcgctgttcttcggagacacgggcttgtcaaagaatcccatcgagctggtggaaggatggttttcatcctggaaa
agcagcatcgcttcattcttcttcatcattggcctgatcatcggcctatttctagtcctgcgggtgggaattcatct
gtgcatcaagctcaagcacactaagaagcggcaaatctacactgatatcgagatgaatcgcctgggcaag
**SEQ ID NO: 4 - VSV-G amino acid** 
MKCLLYLAFLFIGVNCKFTIVFPHNQKGNWKNVPSNYHYCPSSSDLNWHNDLIGTALQVKMPKSHKAIQADGWMCHA
SKWVTTCDFRWYGPKYITHSIRSFTPSVEQCKESIEQTKQGTWLNPGFPPQSCGYATVTDAEAVIVQVTPHHVLVDE
YTGEWVDSQFINGKCSNYICPTVHNSTTWHSDYKVKGLCDSNLISMDITFFSEDGELSSLGKEGTGFRSNYFAYETG
GKACKMQYCKHWGVRLPSGVWFEMADKDLFAAARFPECPEGSSISAPSQTSVDVSLIQDVERILDYSLCQETWSKIR
AGLPISPVDLSYLAPKNPGTGPAFTIINGTLKYFETRYIRVDIAAPILSRMVGMISGTTTERELWDDWAPYEDVEIG
PNGVLRTSSGYKFPLYMIGHGMLDSDLHLSSKAQVFEHPHIQDAASQLPDDESLFFGDTGLSKNPIELVEGWFSSWK
SSIASFFFIIGLIIGLFLVLRVGIHLCIKLKHTKKRQIYTDIEMNRLGK
**SEQ ID NO: 5 - HIV-1 *gag-pol* nucleotide** 
atgggtgcgagagcgtcagtattaagcgggggagaattagatcgatgggaaaaaattcggttaaggccagggggaaa
gaaaaaatataaattaaaacatatagtatgggcaagcagggagctagaacgattcgcagttaatcctggcctgttag
aaacatcagaaggctgtagacaaatactgggacagctacaaccatcccttcagacaggatcagaagaacttagatca
ttatataatacagtagcaaccctctattgtgtgcatcaaaggatagagataaaagacaccaaggaagctttagacaa
gatagaggaagagcaaaacaaaagtaagaaaaaagcacagcaagcagcagctgacacaggacacagcaatcaggtca
gccaaaattaccctatagtgcagaacatccaggggcaaatggtacatcaggccatatcacctagaactttaaatgca
tgggtaaaagtagtagaagagaaggctttcagcccagaagtgatacccatgttttcagcattatcagaaggagccac
 
cccacaagatttaaacaccatgctaaacacagtggggggacatcaagcagccatgcaaatgttaaaagagaccatca
atgaggaagctgcagaatgggatagagtgcatccagtgcatgcagggcctattgcaccaggccagatgagagaacca
aggggaagtgacatagcaggaactactagtacccttcaggaacaaataggatggatgacacataatccacctatccc
agtaggagaaatctataaaagatggataatcctgggattaaataaaatagtaagaatgtatagccctaccagcattc
tggacataagacaaggaccaaaggaaccctttagagactatgtagaccgattctataaaactctaagagccgagcaa
gcttcacaagaggtaaaaaattggatgacagaaaccttgttggtccaaaatgcgaacccagattgtaagactatttt
aaaagcattgggaccaggagcgacactagaagaaatgatgacagcatgtcagggagtggggggacccggccataaag
caagagttttggctgaagcaatgagccaagtaacaaatccagctaccataatgatacagaaaggcaatttaggaac
caaagaaagactgttaagtgtttcaattgtggcaaagaagggcacatagccaaaaattgcagggcccctaggaaaaa
gggctgttggaaatgtggaaaggaaggacaccaaatgaaagattgtactgagagacaggctaattttttagggaaga
tctggccttcccacaaggaaggccagggaatttcttcagagcagaccagagccaacagccccaccagaagagagc
ttcaggtttggggaagagacaacaactccctctcagaagcaggagccgatagacaaggaactgtatcctttagcttc
cctcagatcactctttggcagcgacccctcgtcacaataaagataggggggcaattaaaggaagctctattagatac
aggagcagatgatacagtattagaagaaatgaatttgccaggaagatggaaaccaaaaatgataggggggaattggag
gttttatcaaagtaagacagtatgatcagatactcatagaaatctgcggacataaagctataggtacagtattagta
ggacctacacctgtcaacataattggaagaaatctgttgactcagattggctgcactttaaattttcccattagtcc
tattgagactgtaccagtaaaattaaagccaggaatggatggcccaaaagttaaacaatggccattgacagaagaaa
aaataaaagcattagtagaaatttgtacagaaatggaaaaggaaggaaaaatttcaaaaattgggcctgaaaatcca
tacaatactccagtatttgccataaagaaaaaagacagtactaaatggagaaaattagtagatttcagagaacttaa
taagagaactcaagatttctgggaagttcaattaggaataccacatcctgcagggttaaaacagaaaaaatcagtaa
cagtactggatgtgggcgatgcatatttttcagttcccttagataaagacttcaggaagtatactgcatttaccata
cctagtataaacaatgagacaccagggattagatatcagtacaatgtgcttccacagggatggaaaggatcaccagc
aatattccagtgtagcatgacaaaaatcttagagccttttagaaaacaaaatccagacatagtcatctatcaataca
tggatgatttgtatgtaggatctgacttagaaatagggcagcatagaacaaaaatagaggaactgagacaacatctg
ttgaggtggggatttaccacaccagacaaaaaacatcagaaagaacctccattcctttggatgggttatgaactcca
tcctgataaatggacagtacagcctatagtgctgccagaaaaggacagctggactgtcaatgacatacagaaattag
tgggaaaattgaattgggcaagtcagatttatgcagggattaaagtaaggcaattatgtaaacttcttaggggaacc
aaagcactaacagaagtagtaccactaacagaagaagcagagctagaactggcagaaaacagggagattctaaaaga
accggtacatggagtgtattatgacccatcaaaagacttaatagcagaaatacagaagcaggggcaaggccaatgga
catatcaaatttatcaagagccatttaaaaatctgaaaacaggaaagtatgcaagaatgaagggtgcccacactaat
gatgtgaaacaattaacagaggcagtacaaaaaatagccacagaaagcatagtaatatggggaaagactcctaaatt
taaattacccatacaaaaggaaacatggaagcatggtggacagagtattggcaagccacctggattcctgagtggg
agtttgtcaatacccctcccttagtgaagttatggtaccagttagagaaagaacccataataggagcagaaactttc
tatgtagatggggcagccaataggaaactaaattaggaaaagcaggatatgtaactgacagaggaagacaaaaagt
tgtcccccctaacggacacaacaaatcagaagactgagttacaagcaattcatctagctttgcaggattcgggattag
aagtaaacatagtgacagactcacaatatgcattgggaatcattcaagcacaaccagataagagtgaatcagagtta
gtcagtcaaataatagagcagttaataaaaaaggaaaaagtctacctggcatgggtaccagcacacaaaggaattgg
aggaaatgaacaagtagataaattggtcagtgctggaatcaggaaagtactattttagatggaatagataaggccc
aagaagaacatgagaaatatcacagtaattggagagcaatggctagtgattttaacctaccacctgtagtagcaaaa
 
gaaatagtagccagctgtgataaatgtcagctaaaagggaagccatgcatggacaagtagactgtagcccaggaat
atggcagctagattgtacacatttagaaggaaaagttatcttggtagcagttcatgtagccagtggatatatagaag
cagaagtaattccagcagagacagggcaagaaacagcatacttcctcttaaaattagcaggaagatggccagtaaaa
acagtacatacagacaatggcagcaatttcaccagtactacagttaaggccgcctgttggtgggcggggatcaagca
ggaatttggcattccctacaatccccaaagtcaaggagtaatagaatctatgaataaagaattaaagaaaattatag
gacaggtaagagatcaggctgaacatcttaagacagcagtacaaatggcagtattcatccacaattttaaaagaaaa
ggggggattggggggtacagtgcaggggaaagaatagtagacataatagcaacagacatacaaactaaagaattaca
aaaacaaattacaaaaattcaaaattttcgggtttattacagggacagcagagatccagtttggaaaggaccagcaa
agctcctctggaaaggtgaaggggcagtagtaatacaagataatagtgacataaaagtagtgccaagaagaaaagca
aagatcatcagggattatggaaaacagatggcaggtgatgattgtgtggcaagtagacaggatgaggattaa
**SEQ ID NO: 6 - HIV-1 *rev* nucleotide** 
ATGGCAGGCCGCTCAGGGACTCGGATGAGGATCTGCTGAAGGCGGTGCGGCTCATCAAATTCCTGTACCAGAGCAA
CCCGCCACCGAACCCCGAAGGAACTCGCCAGGCTCGCAGGAACCGCCGCAGACGCTGGCGCGAACGGCAGCGCCAGA
TCCACAGCATCAGCGAACGCATCCTGTCAACTTACTTGGGACGGTCAGCGGAACCTGTCCCGCTGCAGCTGCCGCCG
CTGGAGCGCCTGACTCTGGATTGCAACGAAGACTGCGGAACCAGCGGAACCCAGGGCGTGGGAAGCCCACAGATCCT
GGTGGAATCGCCTACCATCTTGGAAAGCGGCGCGAAAGAA
**SEQ ID NO: 7 - HIV-1 Rev amino acid** 
MAGRSGDSDEDLLKAVRLIKFLYQSNPPPNPEGTRQARRNRRRRWRERQRQIHSISERILSTYLGRSAEPVPLQLPP
LERLTLDCNEDCGTSGTQGVGSPQILVESPTILESGAKE
**SEQ ID NO: 8 - TetR binding site**
   tccctatcagtgatagaga
**SEQ ID NO: 9 - nucleotide sequence of the TetR protein** 
Atgtcgcgcctggacaaaagcaaagtgattaactcagcgctggaactgttgaatgaggtgggaattgaaggactcac
tactcgcaagctggcacagaagctgggcgtcgagcagccaacgctgtactggcatgtgaagaataaacgggcgctcc
tagacgcgcttgccatcgaaatgctggaccgccatcacacccactttgcccctggaggcgaatcctggcaagat
tttctgcggaacaatgcaaagtcgttccggtgcgctctgctgtcccaccgcgatggcgcaaaagtgcacctgggcac
tcggcccaccgagaaacaatacgaaaccctggaaaaccaactggctttcctttgccaacagggattttcactggaga
atgccctgtacgcactatccgcggtcggccactttaccctgggatgcgtcctcgaagatcaggagcaccaagtcgcc
aaggaggaaagagaaactcctaccactgactcaatgcctccgctcctgagacaagccatcgagctgttcgaccacca
gggtgctgaacctgcatttctgttcgggcttgaactgattatctgcggcctggagaaacagttgaagtgcgagtcgg
gatcctag
**SEQ ID NO: 10 - amino acid sequence of the TetR protein** 
MSRLDKSKVINSALELLNEVGIEGLTTRKLAQKLGVEQPTLYWHVKNKRALLDALAIEMLDRHHTHFCPLEGESWQD
FLRNNAKSFRCALLSHRDGAKVHLGTRPTEKQYETLENQLAFLCQQGFSLENALYALSAVGHFTLGCVLEDQEHQVA
KEERETPTTDSMPPLLRQAIELFDHQGAEPAFLFGLELIICGLEKQLKCESGS
**SEQ ID NO: 11 - Apoptosis Inhibitor IAP1** 
ATGAACGAGGACACTCCGCCGTTTATTTATCAATACGCGACAACTTTCGCGATAACATCGCCGAACACGTATT
CGATATGTTACTAGAAAGACATGGCTCGTTTGAAAATTATCCCATTGTAAACACGGCATTCATCAACAGCTTGATCG
TTAACGGGTTTAAATACAATCAAGTCGATGACCACGTTGTGTGCGAGTATTGTGAAGCAGAAATAAAAAATTGGTCC
GAAGACGAGTGTATTGAATATGCACACGTAACCTTGTCGCCGTATTGCGCCTACGCCAATAAGATTGCTGAGCATGA
ATCGTTTGGCGACAACATTACCATCAACGCTGTACTGGTAAAAGAAGGCAGACCCAAGTGTGTGTACAGATGCATGT
CCAATTTACAGTCGCGTATGGATACGTTTGTTACTTTTTGGCCTGCCGCATTGCGTGACATGATTATAAACATCGCG
GAAGCGGGACTTTTTTACACGGGTCGCGGAGACGAAACTGTATGTTTCTTTTGCGATTGTTGCGTACGTGATTGGCA
TACTAACGAAGACGCCTGGCAGCGACACGCCACCGAAAACCCGCAATGCTACTTTGTGCTGTCGGTGAAAGGTAAAG
AATTTTGTCAAAACGCAATTACTGCCACTCACGTTGATAAACGTGACGATGACGACGACGATGATAATTTAAAC
GAGAGCGTCGATGACATTGAGGAAAAATACGAATGCAAAGTCTGTCTTGAACGCCAACGCGACGCAGTGCTTATGCC
TTGTCGGCATTTTTGTGTTTGCGTTCAGTGTTATTTTGGTTTAGATCAAAAGTGTCCGACCTGTCGTCAAGACGTCA
CCGATTTCATAAAAATATTTGTGGTGTAG
**SEQ ID NO: 12 - Apoptosis Inhibitor EBNA5** 
ATGGGAGATCGTAGCGAAGTCCCCGGTCCGGCACGCCCCGGACCTCCGGGAATTGGCCCCGAAGGCCCTCTAGGACA
GCTCCTGCGTCGGCACCGCAGTCCCTCCCCGACCCGTGGAGGCCAAGAGCCCCGGCGGGTCAGACGCCGCGTATTAG
TCCAGCAGGAAGAGGAAGTAGTAAGTGGCTCACCATCAGGGCCCCGGGGAGACAGGTCAGAGGTCCCAGGCCCAGCC
CGCCCTGGCCCGCCGGGTATCGGACCCGAAGGGCCCCTGGGCCAGCTGTTGCGCCGGCACAGATCACCCAGCCCCAC
CCGCGGCGGTCAGGAACCTCGCCGGGTCAGACGGCGGGTGCTCGTACAACAGGAAGAGGAAGTTGTTTCTGGATCGC
CCTCGGGCCCGCGCGGCGACCGCTCAGAGGTGCCTGGACCAGCCCGGCCTGGGCCCCCGGAATCGGACCTGAAGGA
CCGCTGGGTCAGTTACTACGCCGGCACCGGTCCCCTTCGCCGACTCGGGGCGGGCAGGAACCCCGGCGCGTGAGGCG
TCGCGTCCTGGTCCAGCAGGAGGAAGAGGTTGTCAGCGGCAGCCCATCCGGGCCGAGGGGGGATCGTTCGGAAGTGC
CCGGCCCAGCACGCCCGGGCCCGCCAGGTATTGGGCCCGAAGGTCCGTTAGGTCAGCTGCTCCGGCGGCATAGGTCA
CCATCCCCGACTCGGGGCGGCCAGGAACCGCGGAGAGTGCGCCGGAGAGTGCTGGTGCAACAGGAGGAAGAAGTCGT
GTCCGGGTCGCCGTCAGGTCCTCGGGGCGACCGGTCAGAAGTACCTGGACCGGCCCGCCCCGGACCGCCGGGCATCG
GGCCGGAAGGCCCCCTGGGACAGCTGCTGCGGAGACATAGATCGCCATCCCCCACCAGAGGCGGACAGGAACCGCGC
CGCGTGCGCCGCCGGGTGCTGGTTCAGCAAGAAGAAGAGGTTGTGTCGGGTTCACCTAGCGGCCCGAGAGGCGACCG
GAGCGAAGTGCCAGGACCAGCACGTCCGGGCCCTCCAGGTATCGGCCCAGAAGGACCACTGGGACAACTGCTGAGAC
GCCATCGCTCGCCGAGCCCTACGCGCGGAGGTCAAGAACCGAGACGGGTCCGCAGACGAGTCCTCGTTCAACAAGAA
GAAGAGGTCGTGTCTGGAAGCCCGTCTGGCCCAAGAGGGGACAGAAGCGAGGTGCCGGGACCGGCGCGGCCGGGGCC
GCCGGGGATCGGGCCTGAAGGTCCGCTGGGGCAGCTCTTGCGCAGACACCGCTCGCCCAGCCCAACCCGCGGTGGAC
AAGAACCCCGACGGGTGCGGCGGCGCGTGCTCGTGCAACAAGAAGAAGAGGTTGTCTCGGGCTCGCCATCTGGCCCG
 
CTCAGACCAAGACCGCGACCGCCGGCCCGGTCCCTCCGCGAATGGCTGCTGCGCATCAGAGACAGATTCGAGCCGCC
AACTGAAACCACCCAGCGGCAGTCCATCTACATTGAGGAAGAGGAAGATGAGGATTAG
**SEQ ID NO: 13 - Apoptosis Inhibitor BCL-XL** 
ATGAGCCAGTCAAATCGGGAACTGGTGGTGGATTTTCTGAGCTACAAGCTCTCGCAAAAGGGCTACTCATGGAGCCA
GTTTTCGGATGTCGAAGAAAACCGGACCGAGGCTCCAGAGGGCACCGAATCGGAGATGGAAACTCCGTCAGCAATCA
ACGGAAATCCATCATGGCACCTGGCAGATAGCCCGGCGGTGAACGGAGCAACCGGACATTCAAGCTCCCTGGACGCC
AGAGAAGTGATTCCGATGGCGGCAGTGAAGCAGGCGCTACGCGAAGCGGGAGACGAGTTCGAGCTGCGGTACAGGAG
AGCTTTTAGCGACCTGACTAGCCAGCTCCACATCACTCCGGGGACCGCCTACCAGTCGTTTGAACAGGTGGTGAACG
AGCTGTTTCGGGATGGAGTCAACTGGGGCAGAATCGTGGCCTTCTTTTCCTTCGGCGGTGCGCTGTGCGTCGAATCC
GTGGACAAGGAGATGCAGGTCCTGGTCAGCCGGATCGCAGCGTGGATGGCCACTTATCTCAACGATCACCTGGAGCC
GTGGATTCAAGAGAATGGGGGCTGGGACACCTTCGTGGAACTGTATGGAAACAACGCGGCAGCAGAGTCGAGGAAGG
GCCAAGAACGCTTTAATCGGTGGTTCCTGACTGGAATGACGGTGGCAGGAGTGGTGCTACTGGGCTCGCTTTTCAGC
CGCAAATAA
**SEQ ID NO: 14 - N-terminal signal peptide from VSV-G**
   MLSYLIFALAVSPILG

## Claims

1. A retroviral vector comprising a double-stranded nucleic acid molecule comprising:
(a) a first nucleic acid comprising a viral *env* gene; and
(b) a second nucleic acid comprising a viral *gag-pol* gene;
wherein the coding sequences of the first and second nucleic acids are on opposing strands of the double-stranded nucleic acid molecule, wherein the *env* and *gag-pol* genes are independently operably-associated with first and second inducible promoters, respectively, the first and second promoters having less than 95% nucleotide sequence identity between them, and wherein the double-stranded nucleic acid molecule comprises nucleotide sequences encoding apoptosis inhibitors IAP1 and EBNA5.

2. A retroviral vector as claimed in claim 1, wherein the nucleotide sequence identity between the first and second promoters is less than 90%, 85%, 80%, 70% or 60%.

3. A retroviral vector as claimed in claim 1 or claim 2, wherein:
(a) the *env* gene is a *VSV-G* gene; and/or
(b) the *gag-pol* gene is from HIV-1, or is a derivative thereof; and/or
(c) the nucleic acid molecule additionally comprises a *rev* gene; and/or
(d) the nucleic acid molecule additionally comprises a transgene.

4. A retroviral vector as claimed in any one of the preceding claims, wherein the retroviral vector is a lentiviral vector.

5. A mammalian cell comprising a retroviral vector as claimed in any one of claims 1 to 3, preferably wherein the retroviral vector is stably integrated into the nuclear genome of the mammalian cell.

6. A retroviral packaging cell comprising a retroviral vector as claimed in any one of claims 1 to 3.

7. A kit comprising:
(i) a retroviral packaging vector comprising a retroviral vector as claimed in any one of claims 1 to 3,
together with one or more of the following:
(ii) a retroviral Transfer Vector comprising a transgene and a retroviral *rev* gene;
(iii) cells of a cell line suitable for the production of virus particles.

8. A kit comprising:
(i) a retroviral packaging vector comprising a retroviral vector as claimed in any one of claims 1 to 3 which additionally comprises a *rev* gene,
together with one or more of the following:
(ii) a retroviral Transfer Vector comprising a transgene;
(iii) cells of a cell line suitable for the production of virus particles.

9. A process for producing a retroviral packaging cell, the process comprising the steps:
(i) stably integrating a retroviral vector as claimed in any one of claims 1 to 3 into a mammalian cell,
thereby producing a mammalian cell that expresses retroviral *env* and *gag-pol* genes, and optionally the *rev* gene.

10. Use of a retroviral packaging cell as claimed in claim 6 in the production of a retrovirus particle.

11. A process for producing retroviruses, the process comprising the steps:
(a) introducing a retroviral Transfer Vector comprising 5' and 3' retrovirus LTRs, a retrovirus packaging signal and a retroviral *rev* gene into a retroviral packaging cell as claimed in claim 6, wherein the retroviral packaging cell comprises retroviral *env* and *gag-pol* genes stably integrated into its genome;
(b) culturing the cell under conditions such that retroviruses are assembled and secreted by the cell; and
(c) harvesting packaged retrovirus from the supernatant.

12. A process for producing retroviruses, the process comprising the steps:
(a) introducing a retroviral Transfer Vector comprising a transgene into a retroviral packaging cell as claimed in claim 6, wherein the retroviral packaging cell comprises retroviral *env, gag-pol* and *rev* genes stably integrated into its genome;
(b) culturing the cell under conditions such that retroviruses are assembled and secreted by the cell; and
(c) harvesting packaged retrovirus from the supernatant.

## Patentansprüche

1. Retroviraler Vektor, umfassend ein doppelsträngiges Nukleinsäuremolekül, umfassend:
(a) eine erste Nukleinsäure, die ein virales *env*-Gen umfasst; und
(b) eine zweite Nukleinsäure, die ein *virales gag-pol*-Gen umfasst;
wobei sich die Kodierungssequenzen der ersten und der zweiten Nukleinsäure auf gegenüberliegenden Strängen des doppelsträngigen Nukleinsäuremoleküls befinden, wobei die *env*- und *gag-pol*-Gene unabhängig voneinander jeweils operativ mit einem ersten und einem zweiten induzierbaren Promotor assoziiert sind, wobei der erste und der zweite Promotor eine Nukleotidsequenzidentität von weniger als 95 % zwischen ihnen aufweisen, und wobei das doppelsträngige Nukleinsäuremolekül Nukleotidsequenzen umfasst, die für die Apoptose-Inhibitoren IAP1 und EBNA5 kodieren.

2. Retroviraler Vektor nach Anspruch 1, wobei die Nukleotidsequenzidentität zwischen dem ersten und dem zweiten Promotor weniger als 90 %, 85 %, 80 %, 70 % oder 60 % beträgt.

3. Retroviraler Vektor nach Anspruch 1 oder Anspruch 2, wobei:
(a) das *env*-Gen ein VSV-G-Gen ist; und/oder
(b) das *gag-pol*-Gen von HIV-1 stammt oder ein Derivat davon ist; und/oder
(c) das Nukleinsäuremolekül zusätzlich ein *rev*-Gen umfasst; und/oder
(d) das Nukleinsäuremolekül zusätzlich ein Transgen umfasst.

4. Retroviraler Vektor nach einem der vorstehenden Ansprüche, wobei der retrovirale Vektor ein lentiviraler Vektor ist.

5. Säugetierzelle, die einen retroviralen Vektor nach einem der Ansprüche 1 bis 3 umfasst, wobei bevorzugt der retrovirale Vektor stabil in das Kerngenom der Säugetierzelle integriert ist.

6. Retrovirale Verpackungszelle, die einen retroviralen Vektor nach einem der Ansprüche 1 bis 3 umfasst.

7. Satz, umfassend:
(i) einen retroviralen Verpackungsvektor, der einen retroviralen Vektor nach einem der Ansprüche 1 bis 3 umfasst,
zusammen mit einem oder mehreren von Folgendem:
(ii) einen retroviralen Transfervektor, der ein Transgen und ein retrovirales *rev*-Gen umfasst;
(iii) Zellen einer Zelllinie, die für die Produktion von Viruspartikeln geeignet ist.

8. Satz, umfassend:
(i) einen retroviralen Verpackungsvektor, der einen retroviralen Vektor nach einem der Ansprüche 1 bis 3 umfasst, der zusätzlich ein *rev*-Gen umfasst,
zusammen mit einem oder mehreren von Folgendem:
(ii) einen retroviralen Transfervektor, der ein Transgen umfasst;
(iii) Zellen einer Zelllinie, die für die Produktion von Viruspartikeln geeignet ist.

9. Verfahren zur Herstellung einer retroviralen Verpackungszelle, wobei das Verfahren die Schritte umfasst:
(i) stabiles Integrieren eines retroviralen Vektors nach einem der Ansprüche 1 bis 3 in eine Säugetierzelle,
wodurch eine Säugetierzelle hergestellt wird, die retroviralen *env*- und *gag-pol*-Gene und wahlweise das *rev*-Gen exprimiert.

10. Verwendung einer retroviralen Verpackungszelle nach Anspruch 6 bei der Herstellung eines Retroviruspartikels.

11. Verfahren zur Herstellung von Retroviren, wobei das Verfahren die Schritte umfasst:
(a) Einführen eines retroviralen Transfervektors, der 5'- und 3'-Retrovirus-LTRs, ein Retrovirus-Verpackungssignal und ein retrovirales *rev*-Gen umfasst, in eine retrovirale Verpackungszelle nach Anspruch 6, wobei die retrovirale Verpackungszelle retrovirale *env*- und *gag-pol*-Gene umfasst, die stabil in ihr Genom integriert sind;
(b) Kultivieren der Zelle unter Bedingungen, unter denen Retroviren von der Zelle gebildet und ausgeschieden werden; und
(c) Entnehmen verpackter Retroviren aus dem Überstand.

12. Verfahren zur Herstellung von Retroviren, wobei das Verfahren die Schritte umfasst:
(a) Einführen eines retroviralen Transfervektors, der ein Transgen umfasst, in eine retrovirale Verpackungszelle nach Anspruch 6, wobei die retrovirale Verpackungszelle retrovirale env-, *gag-pol*- und *rev*-Gene umfasst, die stabil in ihr Genom integriert sind;
(b) Kultivieren der Zelle unter Bedingungen, bei denen Retroviren gebildet und von der Zelle ausgeschieden werden; und
(c) Entnehmen verpackter Retroviren aus dem Überstand.

## Revendications

1. Vecteur rétroviral comprenant une molécule d'acide nucléique à double brin comprenant :
(a) un premier acide nucléique comprenant un gène viral *env* ; et
(b) un deuxième acide nucléique comprenant un gène *viral gag-pol* ;
dans lequel les séquences codantes des premier et deuxième acides nucléiques se situent sur des brins opposés de la molécule d'acide nucléique à double brin, dans lequel les gènes *env* et *gag-pol* sont indépendamment associés de manière fonctionnelle à des premier et deuxième promoteurs inductibles, respectivement, les premier et deuxième promoteurs présentant une identité de séquence nucléotidique inférieure à 95 % entre eux, et dans lequel la molécule d'acide nucléique à double brin comprend des séquences nucléotidiques codant pour des inhibiteurs d'apoptose IAP1 et EBNA5.

2. Vecteur rétroviral selon la revendication 1, dans lequel l'identité de séquence nucléotidique entre les premier et deuxième promoteurs est inférieure à 90 %, 85 %, 80 %, 70 % ou 60 %.

3. Vecteur rétroviral selon la revendication 1 ou la revendication 2, dans lequel :
(a) le gène *env* est un gène VSV-G ; et/ou
(b) le gène *gag-pol* provient du VIH-1, ou est un dérivé de celui-ci ; et/ou
(c) la molécule d'acide nucléique comprend en outre un gène *rev* ; et/ou
(d) la molécule d'acide nucléique comprend en outre un transgène.

4. Vecteur rétroviral selon l'une quelconque des revendications précédentes, dans lequel le vecteur rétroviral est un vecteur lentiviral.

5. Cellule de mammifère comprenant un vecteur rétroviral selon l'une quelconque des revendications 1 à 3, préférablement dans laquelle le vecteur rétroviral est intégré de manière stable dans le génome nucléaire de la cellule de mammifère.

6. Cellule d'encapsidation rétrovirale comprenant un vecteur rétroviral selon l'une quelconque des revendications 1 à 3.

7. Kit comprenant :
(i) un vecteur d'encapsidation rétrovirale comprenant un vecteur rétroviral selon l'une quelconque des revendications 1 à 3,
conjointement avec un ou plusieurs des éléments suivants :
(ii) un vecteur de transfert rétroviral comprenant un transgène et un gène *rev* rétroviral ;
(iii) des cellules d'une lignée cellulaire adaptées à la production de particules virales.

8. Kit comprenant :
(i) un vecteur d'encapsidation rétrovirale comprenant un vecteur rétroviral selon l'une quelconque des revendications 1 à 3 qui comprend en outre un gène *rev*, conjointement avec un ou plusieurs des éléments suivants :
(ii) un vecteur de transfert rétroviral comprenant un transgène ;
(iii) des cellules d'une lignée cellulaire adaptées à la production de particules virales.

9. Procédé de production d'une cellule d'encapsidation rétrovirale, le procédé comprenant les étapes suivantes :
(i) l'intégration stable d'un vecteur rétroviral selon l'une quelconque des revendications 1 à 3 dans une cellule de mammifère,
la production de cette manière d'une cellule de mammifère qui exprime les gènes *env* et *gag-pol* rétroviraux, et facultativement le gène *rev*.

10. Utilisation d'une cellule d'encapsidation rétrovirale selon la revendication 6 dans la production d'une particule rétrovirale.

11. Procédé de production de rétrovirus, le procédé comprenant les étapes suivantes :
(a) l'introduction d'un vecteur de transfert rétroviral comprenant des LTR rétroviraux 5' et 3', un signal d'encapsidation de rétrovirus et un gène *rev* rétroviral dans une cellule d'encapsidation rétrovirale selon la revendication 6, dans lequel la cellule d'encapsidation rétrovirale comprend des gènes *env* et *gag-pol* rétroviraux intégrés de manière stable dans son génome ;
(b) la mise en culture de la cellule dans des conditions telles que des rétrovirus sont assemblés et sécrétés par la cellule ; et
(c) la récolte de rétrovirus encapsidés à partir du surnageant.

12. Procédé de production de rétrovirus, le procédé comprenant les étapes suivantes :
(a) l'introduction d'un vecteur de transfert rétroviral comprenant un transgène dans une cellule d'encapsidation rétrovirale selon la revendication 6, dans lequel la cellule d'encapsidation rétrovirale comprend des gènes env, *gag-pol* et *rev* rétroviraux intégrés de manière stable dans son génome ;
(b) la mise en culture de la cellule dans des conditions telles que des rétrovirus sont assemblés ; et sécrétés par la cellule ; et
(c) la récolte de rétrovirus encapsidés à partir du surnageant.
